# EUROPEAN PATENT SPECIFICATION

(11) **EP 1 290 164 B1**
(45) Date of publication and mention of the grant of the patent: **29.11.2006**
(21) Application number: 01924787.3
(22) Date of filing: 06.04.2001
(51) Int. Cl.: C12N 15/12, C12N 5/10, C07K 14/47, C07K 16/18, C12Q 1/68

(54) **HUMAN TWIK-8 MOLECULES AND USES THEREOF**
MENSCHLICHE TWIK-8 MOLEKÜLE UND IHRE VERWENDUNG
MOLECULE TWIK-8 HUMAINE ET LEUR UTILISATIONS

(30) Priority: 07.04.2000 US 195734 P
(43) Date of publication of application: 12.03.2003
(73) Proprietor: Bayer HealthCare AG, 51368 Leverkusen (DE)
(72) Inventor: GLUCKSMANN, Maria, Alexandra, Lexington, MA 02173 (US)
(86) International application number: PCT/US2001/011301
(87) International publication number: WO 2001/077329

(56) References cited:
- WO-A-00/26253
- FINK M ET AL: "A neuronal two P domain K+ channel stimulated by arachidonic acid and polyunsaturated fatty acids" EMBO JOURNAL, OXFORD UNIVERSITY PRESS, SURREY, GB, vol. 17, no. 12, 15 June 1998 (1998-06-15), pages 3297-3308, XP002085600 ISSN: 0261-4189
- LESAGE ET AL: "Cloning and expression of human TRAAK, a polyunsaturated fatty acids-activated and mechano-sensitive K+ channel " FEBS LETTERS, vol. 471, 14 April 2000 (2000-04-14), pages 137-140, XP002190784
- DATABASE EMBL [Online] 17 April 2000 (2000-04-17) GRAY : "Assigment of KCNK4 encoding the human potassium channel TRAAK to chromosome 11 " retrieved from EBI, accession no. AF247042 XP002190785

## Description

### Background of the Invention

Potassium (K⁺) channels are ubiquitous proteins which are involved in the setting of the resting membrane potential as well as in the modulation of the electrical activity of cells. In excitable cells, K⁺ channels influence action potential waveforms, firing frequency, and neurotransmitter secretion (Rudy, B. (1988) *Neuroscience, 25,* 729-749; Hille, B. (1992) *Ionic Channels of Excitable Membranes,* 2nd Ed.). In non-excitable cells, they are involved in hormone secretion, cell volume regulation and potentially in cell proliferation and differentiation (Lewis *et al.* (1995) *Annu. Rev. Immunol.,* 13, 623-653). Developments in electrophysiology have allowed the identification and the characterization of an astonishing variety of K⁺ channels that differ in their biophysical properties, pharmacology, regulation and tissue distribution (Rudy, B. (1988) *Neuroscience,* 25, 729-749; Hille, B. (1992) *Ionic Channels of Excitable Membranes,* 2nd Ed.). More recently, cloning efforts have shed considerable light on the mechanisms that determine this functional diversity. Furthermore, analyses of structure-function relationships have provided an important set of data concerning the molecular basis of the biophysical properties (selectivity, gating, assembly) and the pharmacological properties of cloned K⁺ channels.

Functional diversity of K⁺ channels arises mainly from the existence of a great number of genes coding for pore-forming subunits, as well as for other associated regulatory subunits. Two main structural families of pore-forming subunits have been identified. The first one consists of subunits with a conserved hydrophobic core containing six transmembrane domains (TMDs). These K⁺ channel α subunits participate in the formation of outward rectifier voltage-gated (Kv) and Ca²⁺-dependent K⁺ channels. The fourth TMD contains repeated positive charges involved in the voltage gating of these channels and hence in their outward rectification (Logothetis *et al.* (1992) *Neuron,* 8, 531-540; Bezanilla *et al.* (1994) *Biophys. J.* 66, 1011-102 1).

The second family of pore-forming subunits have only two TMDs. They are essential subunits of inward-rectifying (IRK), G-protein-coupled (GIRK) and ATP-sensitive (K_{ATP}) K⁺ channels. The inward rectification results from a voltage-dependent block by cytoplasmic Mg²⁺ and polyamines (Matsuda, H. (1991) *Annu. Rev. Physiol.,* 53, 289-298). A conserved domain, called the P domain, is present in all members of both families (Pongs, O. (1993) *J. Membr. Biol,* 136, 1-8; Heginbotham *et al.* (1994) *Biophys. J.* 66,1061-1067; Mackinnon, R. (1995) *Neuron,* 14, 889-892; Pascual *et al.,* (1995) *Neuron.,* and 14, 1055-1063). This domain is an essential element of the aqueous K⁺-selective pore. In both groups, the assembly of four subunits is necessary to form a functional K⁺ channel (Mackinnon, R. (1991) *Nature,* 350, 232-235; Yang *et al.,* (1995) *Neuron,* 15, 1441-1447.

In both six TMD and two TMD pore-forming subunit families, different subunits coded by different genes can associate to form heterotetramers with new channel properties (Isacoff *et al.,* (1990) *Nature,* 345, 530-534). A selective formation of heteropolymeric channels may allow each cell to develop the best K⁺ current repertoire suited to its function. Pore-forming α subunits of Kv channels are classified into different subfamilies according to their sequence similarity (Chandy *et al.* (1993) *Trends Pharmacol. Sci.,* 14: 434). Tetramerization is believed to occur preferentially between members of each subgroup (Covarrubias *et al. (1991) Neuron,* 7, 763-773). The domain responsible for this selective association is localized in the N-terminal region and is conserved between members of the same subgroup. This domain is necessary for hetero- but not homo-multimeric assembly within a subfamily and prevents co-assembly between subfamilies. Recently, pore-forming subunits with two TMDs were also shown to co-assemble to form heteropolymers (Duprat *et al.* (1995) *Biochem. Biophys. Res. Commun.,* 212, 657-663. This heteropolymerization seems necessary to give functional GIRKs. IRKs are active as homopolymers but also form heteropolymers.

New structural types of K⁺ channels were identified recently in both humans and yeast. These channels have two P domains in their functional subunit instead of only one (Ketchum *et al.* (1995) *Nature,* 376, 690-695; Lesage *et al.* (1996) *J. Biol. Chem,* 271, 4183-4187; Lesage *et al.* (1996) *EMBO J.,* 15, 1004-1011; Reid *et al.* (1996) *Receptors Channels* 4, 51-62). The human channel called TWIK-1, has four TMDs. TWIK-1 is expressed widely in human tissues and is particularly abundant in the heart and the brain. TWIK-1 currents are time independent and inwardly rectifying. These properties suggest that TWIK-1 channels are involved in the control of the background K⁺ membrane conductance (Lesage *et al.* (1996) *EMBO J.,* 15, 1004-1011). Fink et al, (1998) EMBO J., 17, 3297-3308, report the cloning of TRAAK, a 398 amino acid protein which is a new member of this class of K⁺ channels. WO0026253 discloses h-TRAAK polypeptides and polynucleotides.

### Summary of the Invention

The present invention is based, at least in part, on the discovery of novel members of the TWIK (for Tandem of P domains in a Weak Inward rectifying **K**^{**+**} channel)-like family of potassium channels, referred to herein as TWIK-8 nucleic acid and protein molecules. The TWIK-8 molecules of the present invention are useful as targets for developing modulating agents to regulate a variety of cellular processes. Accordingly, in one aspect, this invention provides isolated nucleic acid molecules encoding TWIK-8 proteins or biologically active portions thereof, as well as nucleic acid fragments suitable as primers or hybridization probes for the detection of TWIK-8-encoding nucleic acids. *e.g.*

In one embodiment, a TWIK-8 nucleic acid molecule of the invention is at least 95%, 96%, 97%, 98%, 99% or more identical to the nucleotide sequence (*e.g.*, to the entire length of the nucleotide sequence) shown in SEQ ID NO:1 or 3, or a complement thereof. In a preferred embodiment, the isolated nucleic acid molecule includes the nucleotide sequence shown SEQ ID NO:1 or 3, or a complement thereof. In another embodiment, the nucleic acid molecule includes SEQ ID NO:3 and nucleotides 1-83 of SEQ ID NO:1. In yet another embodiment, the nucleic acid molecule includes SEQ ID N0:3 and nucleotides 1344-1408 of SEQ ID NO: 1. In another preferred embodiment, the nucleic acid molecule consists of the nucleotide sequence shown in SEQ ID NO:1 or 3.

In another embodiment, a TWIK-8 nucleic acid molecule includes a nucleotide sequence encoding a protein having an amino acid sequence sufficiently identical to the amino acid sequence of SEQ ID NO:2. In a preferred embodiment, a TWIK-8 nucleic acid molecule includes a nucleotide sequence encoding a protein having an amino acid sequence at least 95%, 96%, 97%, 98%, 99% or more identical to the entire length of the amino acid sequence of SEQ ID NO:2.

In another preferred embodiment, an isolated nucleic acid molecule encodes the amino acid sequence of human TWIK-8. In yet another preferred embodiment, the nucleic acid molecule includes a nucleotide sequence encoding a protein having the amino acid sequence of SEQ ID NO:2,.

Another embodiment of the invention features the use of nucleic acid molecules, preferably TWIK-8 nucleic acid molecules, which specifically detect TWIK-8 nucleic acid molecules relative to nucleic acid molecules encoding non-TWIK-8 proteins. For example, in one embodiment, such a nucleic acid molecule is at least 50, 100, 150, 200, 250, 300, 350, 400, 450, 500, 550, 600, 650, 700, 750, 800, 850, 900, 950, 1000, 1050, 1100, 1150, 1200, 1250, 1300, 1350, 1400, 1450, 1500, or more nucleotides in length and hybridizes under stringent conditions to a nucleic acid molecule comprising the nucleotide sequence shown in SEQ ID NO:1, or a complement thereof.

In other preferred embodiments, the nucleic acid molecule encodes a naturally occurring allelic variant of a polypeptide comprising the amino acid sequence of SEQ ID NO:2, wherein the nucleic acid molecule hybridizes to a nucleic acid molecule comprising SEQ ID NO: or 3 under stringent conditions.

Another embodiment of the invention provides an isolated nucleic acid molecule which is antisense to a TWIK-8 nucleic acid molecule, *e.g.*, the coding strand of a TWIK-8 nucleic acid molecule.

Another aspect of the invention provides a vector comprising a TWIK-8 nucleic acid molecule. In certain embodiments, the vector is a recombinant expression vector. In another embodiment, the invention provides a host cell containing a vector of the invention. In yet another embodiment, the invention provides a host cell containing a nucleic acid molecule of the invention. The invention also provides a method for producing a TWIK-8 protein, by culturing in a suitable medium, a host cell, *e.g.*, a mammalian host cell such as a non-human mammalian cell, of the invention containing a recombinant expression vector, such that the protein is produced.

Another aspect of this invention features isolated or recombinant TWIK-8 proteins.

A TWIK-8 protein includes at least one or more of the following domains: a transmembrane domain, a pore loop domain, a seven-transmembrane receptor domain, a cyclic nucleotide-gated channel domain, a TRAAK potassium channel domain, a potassium channel protein domain, a voltage-gated potassium channel domain, a potassium channel subunit domain, and an outward-rectifier TOK1 potassium channel domain, and has an amino acid sequence at least 95%, 96%, 97%, 98%, 99% or more identical to the amino acid sequence of SEQ ID NO:2.

In another embodiment, the invention features a TWIK-8 protein which is encoded by a nucleic acid molecule consisting of a nucleotide sequence at least 95%, 96%, 97%, 98%, 99% or more identical to a nucleotide sequence of SEQ ID NO:1 or 3, or a complement thereof.

The proteins of the present invention, can be operatively linked to a non-TWIK-8 polypeptide (*e.g.*, heterologous amino acid sequences) to form fusion proteins. The invention further features antibodies, such as monoclonal or polyclonal antibodies, that specifically bind proteins of the invention. In addition, the TWIK-8 proteins can be incorporated into pharmaceutical compositions, which optionally include pharmaceutically acceptable carriers.

In another aspect, the present invention provides a method for detecting the presence of a TWIK-8 nucleic acid molecule or protein in a biological sample by contacting the biological sample with an agent capable of detecting a TWIK-8 nucleic acid molecule or protein such that the presence of a TWIK-8 nucleic acid molecule, protein or polypeptide is detected in the biological sample.

In another aspect, the present invention provides a method for detecting the presence of TWIK-8 activity in a biological sample by contacting the biological sample with an agent capable of detecting an indicator of TWIK-8 activity such that the presence of TWIK-8 activity is detected in the biological sample.

In another aspect, the invention provides a method for modulating TWIK-8 activity comprising contacting a cell capable of expressing TWIK-8 with an antibody that modulates TWIK-8 activity such that TWIK-8 activity in the cell is modulated. In one embodiment, the antibody inhibits TWIK.-8 activity. In another embodiment, the antibody stimulates TWIK-8 activity. The antibody is an antibody that specifically binds to a TWIK-8 protein.

In one embodiment, the methods of the present invention are used to treat a subject having a disorder characterized by aberrant or unwanted TWIK-8 protein or nucleic acid expression or activity by administering an antibody which is a TWIK-8 modulator to the subject. The antibody, or an immunologically active portion thereof, selectively binds to the TWIK-8 protein. In a preferred embodiment, the disorder characterized by aberrant or unwanted TWIK-8 protein or nucleic acid expression is a CNS disorder, such as a cognitive or neurodegenerative disorder. In another preferred embodiment, the disorder characterized by aberrant or unwanted TWIK-8 protein or nucleic acid expression is a cardiovascular disorder. In another preferred embodiment, the disorder characterized by aberrant or unwanted TWIK-8 protein or nucleic acid expression is a muscular disorder. In another embodiment, the disorder characterized by aberrant or unwanted TWIK-8 activity is a cell proliferation, growth, differentiation, or migration disorder. In another embodiment, the disorder characterized by aberrant or unwanted TWIK-8 activity is a pain disorder, or a disorder characterized by misregulated pain signaling mechanisms.

The present invention also provides diagnostic assays for identifying the presence or absence of a genetic alteration characterized by at least one of (i) aberrant modification or mutation of a gene encoding a TWIK-8 protein; (ii) mis-regulation of the gene; and (iii) aberrant post-translational modification of a TWIK-8 protein, wherein a wild-type form of the gene encodes a protein with a TWIK-8 activity.

In another aspect the invention provides methods for identifying a compound that binds to or modulates the activity of a TWIK-8 protein, by providing an indicator composition comprising a TWIK-8 protein having TWIK-8 activity, contacting the indicator composition with a test compound, and determining the effect of the test compound on TWIK-8 activity in the indicator composition to identify a compound that modulates the activity of a TWIK-8 protein.

Other features and advantages of the invention will be apparent from the following detailed description and claims.

### Brief Description of the Drawings

*Figure 1* depicts the cDNA sequence and predicted amino acid sequence of human TWIK-8. The nucleotide sequence corresponds to nucleic acids 1 to 1408 of SEQ ID NO:1. The amino acid sequence corresponds to amino acids 1 to 419 of SEQ ID NO: 2. The coding region without the 3' untranslated region of the human TWIK-8 gene is shown in SEQ ID NO: 3.
*Figure 2* depicts the results of an analysis of the human TWIK-8 amino acid sequence (SEQ ID NO:2) by the Signal P program (Henrik, *et al.* (1997) *Protein Engineering* 10:1-6), indicating the presence of a signal peptide at about residues 1-46 of the native molecule.
*Figure 3* depicts the results of a search which was performed against the MEMSAT database and which resulted in the identification of six transmembrane domains in the native human TWIK-8 protein (SEQ ID NO:2), and five transmembrane domains in the mature form of the human TWIK-8 protein.
*Figure 4* depicts the results of a search performed against the HMM database which identified the presence of a "seven-transmembrane receptor domain" and a "cyclic nucleotide-gated channel domain" in the amino acid sequence of human TWIK-8 (SEQ ID NO:2).
*Figure 5* depicts the results of a search performed against the ProDom database which identified the presence of a "TRAAK potassium channel domain", a "potassium channel protein domain", a "voltage-gated potassium channel domain", an "outward-rectifier TOK1 potassium channel domain", and a "potassium channel subunit domain" in the amino acid sequence of human TWIK-8 (SEQ ID NO:2).

### Detailed Description of the Invention

The present invention is based, at least in part, on the discovery of novel molecules, referred to herein as TWIK-8 nucleic acid and protein molecules, which are novel members of the TWIK (for Tandem of P domains in a Weak Inward rectifying **K**^{**+**} channel)-like family of potassium channels. These novel molecules are capable of, for example, modulating a potassium channel mediated activity in a cell, *e.g.*, a neuronal cell, or a muscle cell.

As used herein, a "potassium channel" includes a protein or polypeptide which is involved in receiving, conducting, and transmitting signals in an electrically excitable or a non-electrically excitable cell, *e.g.,* a neuronal cell, or a muscle cell *(e.g.,* a cardiac muscle cell). Potassium channels are potassium ion selective, and can determine membrane excitability (the ability of, for example, a neuron to respond to a stimulus and convert it into an impulse). Potassium channels can also influence the resting potential of membranes, wave forms and frequencies of action potentials, and thresholds of excitation. Potassium channels are typically expressed in electrically excitable cells, *e.g*., neurons, muscle, endocrine, and egg cells, and may form heteromultimeric structures, *e.g*., composed of pore-forming a and cytoplasmic β subunits. Potassium channels may also be found in non-excitable cells (*e.g*., spleen cells or prostate cells), where they may play a role in, *e.g*., signal transduction. Examples of potassium channels include: (1) the voltage-gated potassium channels, (2) the ligand-gated potassium channels, *e.g.,* neurotransmitter-gated potassium channels, and (3) cyclic-nucleotide-gated potassium channels. Voltage-gated and ligand-gated potassium channels are expressed in the brain, *e.g*., in brainstem monoaminergic and forebrain cholinergic neurons, where they are involved in the release of neurotransmitters, or in the dendrites of hippocampal and neocortical pyramidal cells, where they are involved in the processes of learning and memory formation. For a detailed description of potassium channels, see Kandel E.R.. *et al.,* Principles of Neural Science, second edition, (Elsevier Science Publishing Co., Inc., N.Y. (1985)), the contents of which are incorporated herein by reference. As the TWIK-like proteins of the present invention may modulate potassium channel mediated activities, they may be useful for developing novel diagnostic and therapeutic agents for potassium channel associated disorders.

As used herein, a "potassium channel associated disorder" includes a disorder, disease or condition which is characterized by a misregulation of a potassium channel mediated activity. Potassium channel associated disorders can detrimentally affect conveyance of sensory impulses from the periphery to the brain and/or conductance of motor impulses from the brain to the periphery; integration of reflexes; interpretation of sensory impulses; and emotional, intellectual (*e.g.*, learning and memory), or motor processes.

Examples of potassium channel associated disorders include CNS disorders such as cognitive and neurodegenerative disorders, examples of which include, but are not limited to, Alzheimer's disease, dementias related to Alzheimer's disease (such as Pick's disease), Parkinson's and other Lewy diffuse body diseases, senile dementia, Huntington's disease, Gilles de la Tourette's syndrome, multiple sclerosis, amyotrophic lateral sclerosis, movement disorders, progressive supranuclear palsy, epilepsy, AIDS related dementia, and Jakob-Creutzfieldt disease; autonomic function disorders such as hypertension and sleep disorders, and neuropsychiatric disorders, such as depression, schizophrenia, schizoaffective disorder, korsakoff's psychosis, mania, anxiety disorders, or phobic disorders; learning or memory disorders, *e.g.*, amnesia or age-related memory loss, attention deficit disorder, dysthymic disorder, major depressive disorder, mania, obsessive-compulsive disorder, psychoactive substance use disorders, anxiety, phobias, panic disorder, as well as bipolar affective disorder, *e.g.*, severe bipolar affective (mood) disorder (BP-1), and bipolar affective neurological disorders, *e.g.*, migraine and obesity. Further CNS-related disorders include, for example, those listed in the American Psychiatric Association's Diagnostic and Statistical manual of Mental Disorders (DSM), the most current version of which is incorporated herein by reference in its entirety.

Further examples of potassium channel associated disorders include cardiac-related disorders. Cardiovascular system disorders in which the TWIK-8 molecules of the invention may be directly or indirectly involved include arteriosclerosis, ischemia reperfusion injury, restenosis, arterial inflammation, vascular wall remodeling, ventricular remodeling, rapid ventricular pacing, coronary microembolism, tachycardia, bradycardia, pressure overload, aortic bending, coronary artery ligation, vascular heart disease, atrial fibrilation, Jervell syndrome, Lange-Nielsen syndrome, long-QT syndrome, congestive heart failure, sinus node dysfunction, angina, heart failure, hypertension, atrial fibrillation, atrial flutter, dilated cardiomyopathy, idiopathic cardiomyopathy, myocardial infarction, coronary artery disease, coronary artery spasm, and arrhythmia. TWIK-8-mediated or related disorders also include disorders of the musculoskeletal system such as paralysis and muscle weakness, *e.g.*, ataxia, myotonia, and myokymia.

Other examples of potassium channel-associated disorders include pain disorders. Pain disorders include those disorders that affect pain signaling mechanisms. As used herein, the term "pain signaling mechanisms" includes the cellular mechanisms involved in the development and regulation of pain, *e.g.*, pain elicited by noxious chemical, mechanical, or thermal stimuli, in a subject, *e.g.*, a mammal such as a human. In mammals, the initial detection of noxious chemical, mechanical, or thermal stimuli, a process referred to as "nociception", occurs predominantly at the peripheral terminals of specialized, small diameter sensory neurons. These sensory neurons transmit the information to the central nervous system, evoking a perception of pain or discomfort and initiating appropriate protective reflexes. The TWIK-8 molecules of the present invention may be present on these sensory neurons and, thus, may be involved in detecting these noxious chemical, mechanical, or thermal stimuli and transducing this information into membrane depolarization events. Thus, the TWIK-8 molecules by participating in pain signaling mechanisms, may modulate pain elicitadon and act as targets for developing novel diagnostic targets and therapeutic agents to control pain. Examples of pain disorders include headache posttherapeutic neuralgia, diabetic neuropathy, postmastectomy pain syndrome, stump pain, reflex sympathetic dystrophy, trigeminal neuralgia, neuropathic pain, orofacial neuropathic pain, osteoarthritis, arthritis, *e.g.*, rheumatoid arthritis, fibromyalgia syndrome, tension myalgia, Guillian-Barre syndrome, Meralgia paraesthetica, burning mouth syndrome, fibrocitis, myofascial pain syndrome, idiopathic pain disorder, temporomandibular joint syndrome, atypical odontalgia, loin pain, haematuria syndrome, non-cardiac chest pain, back pain, chronic nonspecific pain, pain associated with surgery, psychogenic pain, tooth pain, musculoskeletal pain disorder, chronic pelvic pain, nonorganic chronic headache, tension-type headache, cluster headache, migraine, complex regional pain syndrome, vaginismus, nerve trunk pain, somatoform pain disorder, cyclical mastalgia, chronic fatigue syndrome, multiple somatization syndrome, chronic pain disorder, cancer pain, somatization disorder, Syndrome X, facial pain, idiopathic pain disorder, posttraumatic rheumatic pain modulation disorder (fibrositis syndrome), hyperalgesia, and Tangier disease.

Potassium channel disorders also include cellular proliferation, growth, differentiation, or migration disorders. Cellular proliferation, growth, differentiation, or migration disorders include those disorders that affect cell proliferation, growth, differentiation, or migration processes. As used herein, a "cellular proliferation, growth, differentiation, or migration process" is a process by which a cell increases in number, size or content, by which a cell develops a specialized set of characteristics which differ from that of other cells, or by which a cell moves closer to or further from a particular location or stimulus. The TWIK-8 molecules of the present invention are involved in signal transduction mechanisms, which are known to be involved in cellular growth, differentiation, and migration processes. Thus, the TWIK-8 molecules may modulate cellular growth, differentiation, or migration, and may play a role in disorders characterized by aberrantly regulated growth, differentiation, or migration. Such disorders include cancer, *e.g.*, carcinoma, sarcoma, or leukemia; tumor angiogenesis and metastasis; skeletal dysplasia; neuronal deficiencies resulting from impaired neural induction and patterning; and hematopoietic and/or myeloproliferative disorders.

TWIK-8-associated or related disorders also include disorders of tissues in which TWIK-8 protein is expressed, *e.g.,* cortex, hypothalamus and dorsal root ganglia.

As used herein, a "potassium channel mediated activity" includes an activity which involves a potassium channel, *e.g.*, a potassium channel in a neuronal cell, or a muscle cell (*e.g.*, a cardiac muscle cell), associated with receiving, conducting, and transmitting signals in, for example, the nervous system. Potassium channel mediated activities include release of neurotransmitters, *e.g.,* dopamine or norepinephrine, from cells, *e.g.*, neuronal cells; modulation of resting potential of membranes, wave forms and frequencies of action potentials, and thresholds of excitation; and modulation of processes such as integration of sub-threshold synaptic responses, the conductance of back-propagating action potentials in, for example, neuronal cells or muscle cells, participation in signal transduction pathways, and participation in nociception.

The term "family" when referring to the protein and nucleic acid molecules of the invention is intended to mean two or more proteins or nucleic acid molecules having a common structural domain or motif and having sufficient amino acid or nucleotide sequence homology as defined herein. Such family members can be naturally or non-naturally occurring and can be from either the same or different species. For example, a family can contain a first protein of human origin, as well as other, distinct proteins of human origin or alternatively, can contain homologues of non-human origin, *e.g.*, monkey proteins. Members of a family may also have common functional characteristics.

These family members are not part of this invention unless they fall under the claims.

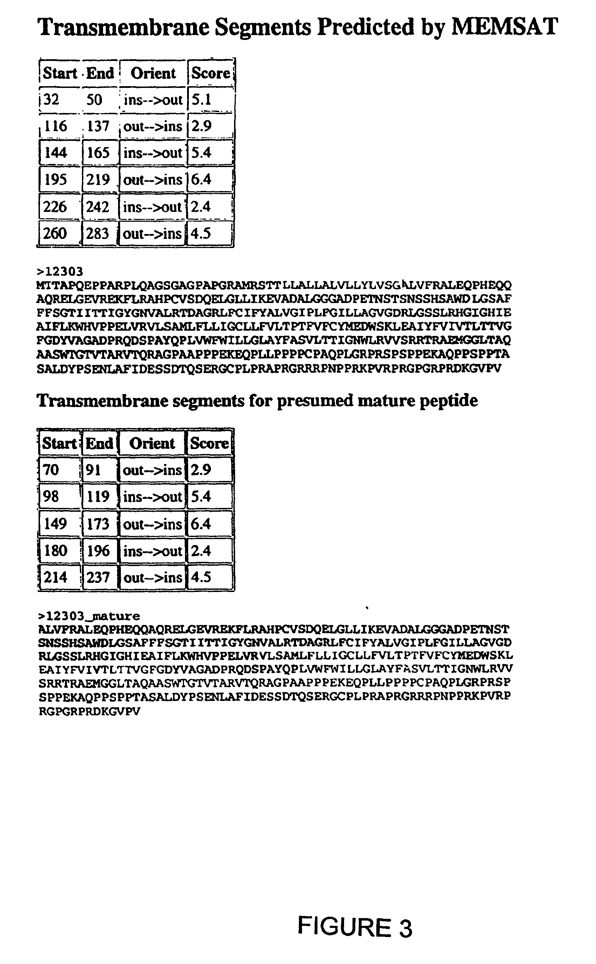

For example, the family of TWBC-8 proteins comprises at least one "transmembrane domain" and preferably six transmembrane domains. As used herein, the term "transmembrane domain" includes an amino acid sequence of about 15 amino acid residues in length which spans the plasma membrane. More preferably, a transmembrane domain includes about at least 20, 25, 30, 35, 40, or 45 amino acid residues and spans the plasma membrane. Transmembrane domains are rich in hydrophobic residues, and typically have an alpha-helical structure. In a preferred embodiment, at least 50%, 60%, 70%, 80%, 90%, 95% or more of the amino acids of a transmembrane domain are hydrophobic, *e.g.*, leucines, isoleucines, tyrosines, or tryptophans. Transmembrane domains are described in, for example, Zagotta W.N. *et al.* (1996) *Annual Rev. Neurosci.* 19: 235-263, the contents of which are incorporated herein by reference. Amino acid residues 32-50, 116-137, 144-165, 195-219, 226-242, and 260-283 of the native TWIK-8 protein, and amino acid residues 70-91, 98-119, 149-173, 180-196 and 214-237 of the mature TWIK-8 protein are predicted to comprise transmembrane domains (see Figure 3).

In another embodiment, a TWIK-8 molecule is identified based on the presence of a Pore loop (P-loop). As used herein, the term "Pore loop" or "P-loop" includes an amino acid sequence of about 15-45 amino acid residues in length, preferably about 15-35 amino acid residues in length, and most preferably about 15-25 amino acid residues in length, which is involved in lining the potassium channel pore. A P-loop is typically found between transmembrane domains of potassium channels and is believed to be a major determinant of ion selectivity in potassium channels. Preferably, P-loops contain a G-[HYDROPHOBIC AMINO ACID]-G sequence, *e.g.,* a GYG, GLG, or GFG sequence. P-loops are described in, for example, Warmke *et al.* (1991) *Science* 252:1560-1562; Zagotta W.N. *et al.,* (1996) *Annual Rev. Neuronsci.* 19:235-63 (Pongs, O. (1993) *J. Membr. Biol.,* 136, 1-8; Heginbotham *et al.* (1994) *Biophys. J* 66,1061-1067; Mackinnon, R. (1995) *Neuron,* and 14, 889-892; Pascual *et al.,* (1995) *Neuron,* 14, 1055-1063). Amino acid residues 243-259 of the native human TWIK-8 protein, and residues 197-213 of the predicted mature human TWIK-8 protein comprise a P-loop.

In a preferred embodiment, the TWIK-8 molecules include at least one and, preferably, six transmembrane domains and at least one P-loop domain.

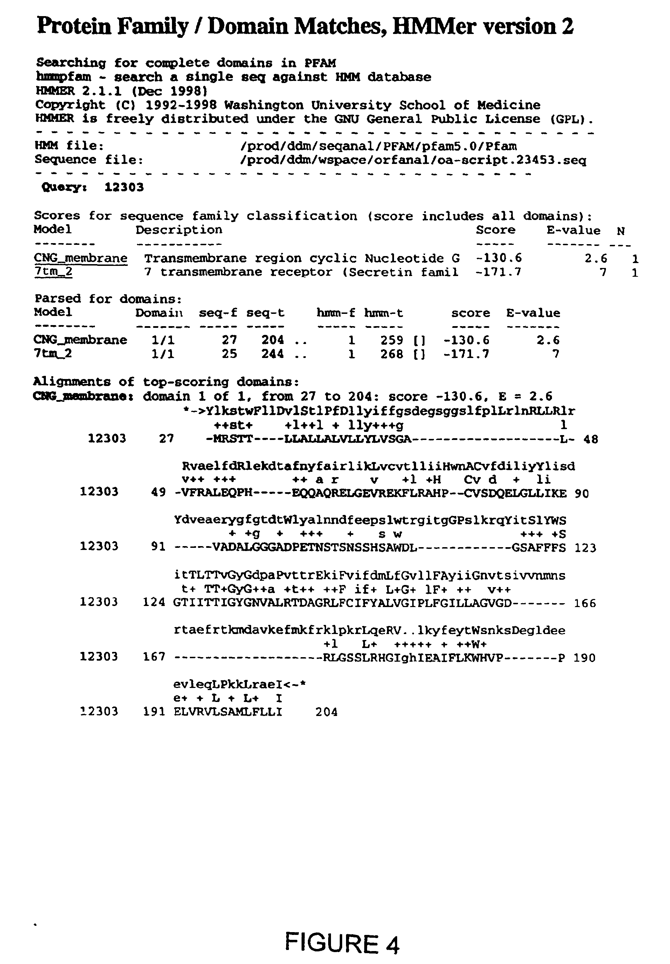

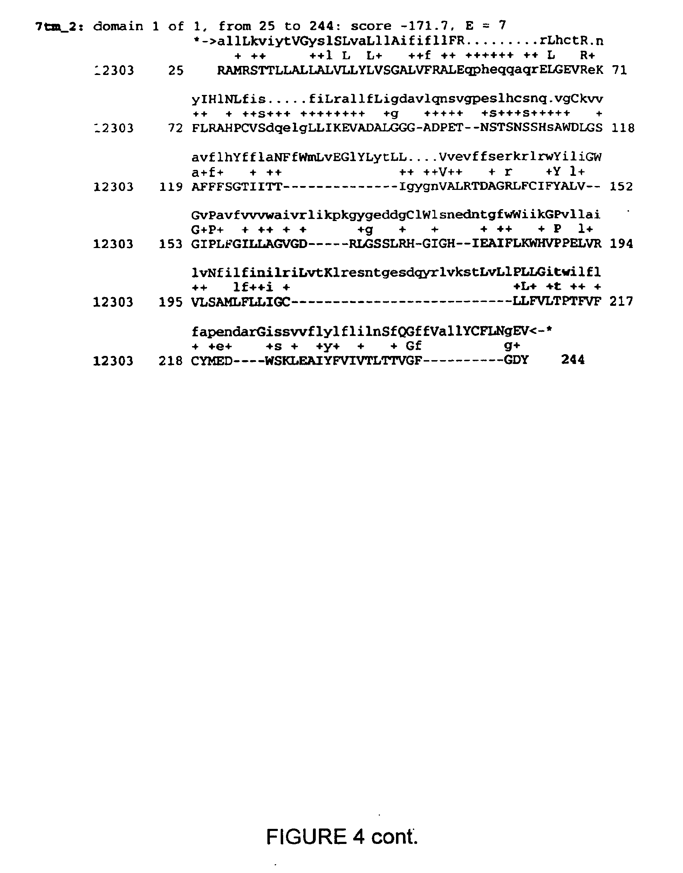

In another embodiment, a TWIK-8 molecule is identified based on the presence of a "seven-transmembrane receptor domain" in the protein or corresponding nucleic acid molecule. Seven-transmembrane receptor domains are described, for example, in Hamann *et al.* (1996) *Genomics* 32: 144-147. As used herein, the term "seven-transmembrane receptor domain" includes a protein domain having an amino acid sequence of about 150-320 amino acid residues. Preferably, a seven-transmembrane receptor domain includes at least about 200-250, or more preferably about 220 amino acid residues. To identify the presence of a seven-transmembrane receptor domain in a TWIK-8 protein, and to make the determination that a protein of interest has a particular profile, the amino acid sequence of the protein is searched against a database of known protein domains (*e.g.*, the HMM database). The seven-transmembrane receptor domain (HMM) has been assigned the PFAM Accession PF00002 (http://genome.wustl.edu/Pfam/html). A search was performed against the HMM database resulting in the identification of a seven-transmembrane receptor domain in the amino acid sequence of human TWIK-8 (SEQ ID NO: 2) at about residues 25-244 of SEQ ID NO: 2. The results of the search are set forth in Figure 4.

A TWIK-8 molecule can be identified based on the presence of a "cyclic nucleotide-gated channel domain" in the protein or corresponding nucleic acid molecule. Cyclic nucleotide-gated channel domains are described, for example, in Yau (1994) *Proc. Natl. Acad Sci. USA* 91: 3481-3483. As used herein, the term "cyclic nucleotide-gated channel domain" includes a protein domain having an amino acid sequence of about 100-225 amino acid residues. Preferably, a cyclic nucleotide-gated channel domain includes at least about 150-200, or more preferably about 178 amino acid residues. To identify the presence of a cyclic nucleotide-gated channel domain in a TWIK-8 protein, and to make the determination that a protein of interest has a particular profile, the amino acid sequence of the protein is searched against a database of known protein domains (*e.g.*, the HMM database). The cyclic nucleotide-gated channel domain (HMM) has been assigned the PFAM Accession PF00914 (http://genome.wustl.edu/Pfam/html). A search was performed against the HMM database resulting in the identification of a cyclic nucleotide-gated channel domain in the amino acid sequence of human TWIK-8 (SEQ ID NO: 2) at about residues 27-204 of SEQ ID NO: 2. The results of the search are set forth in Figure 4.

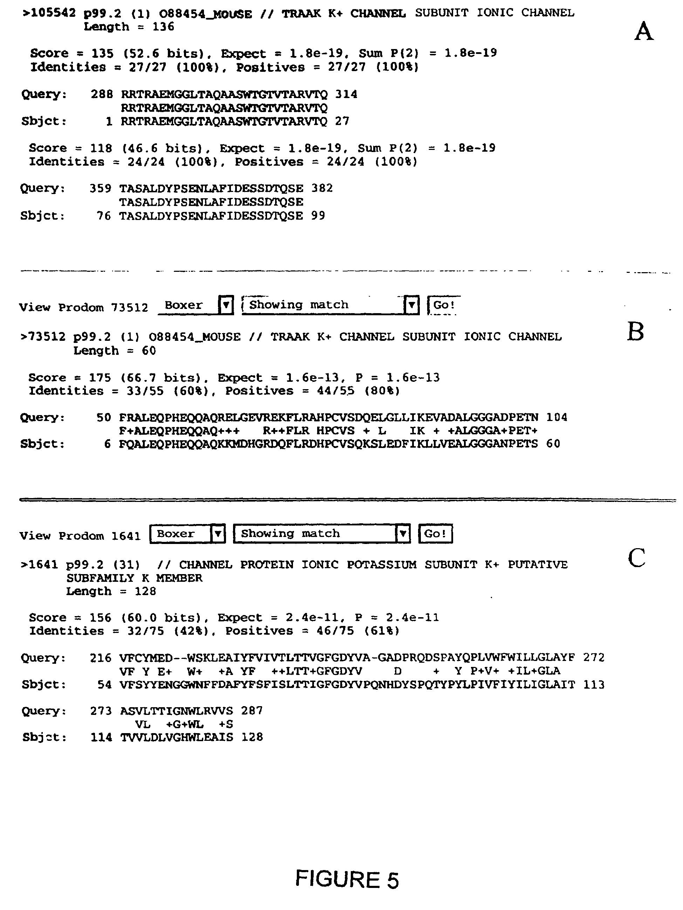

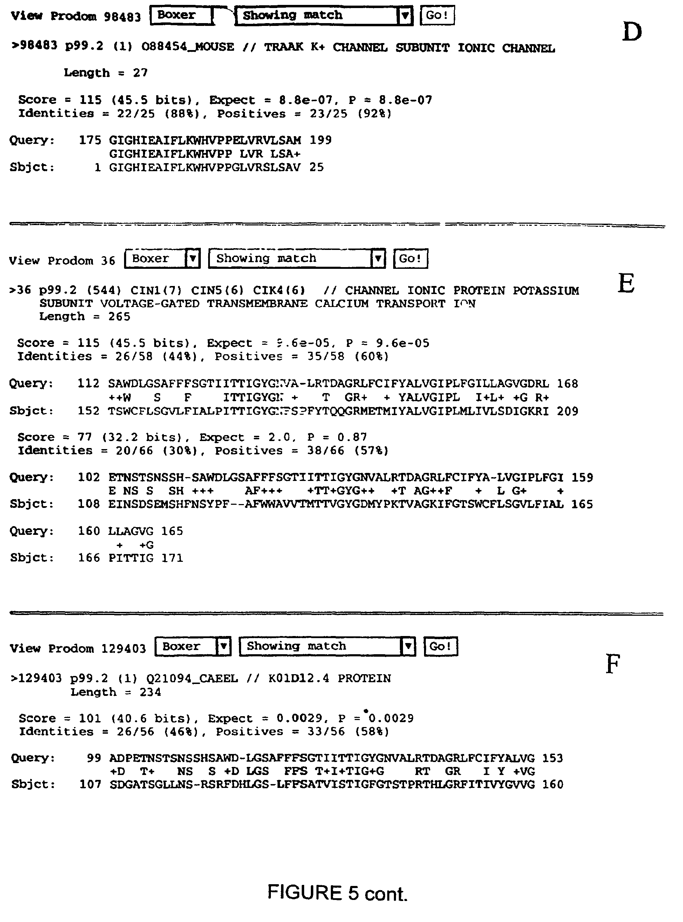

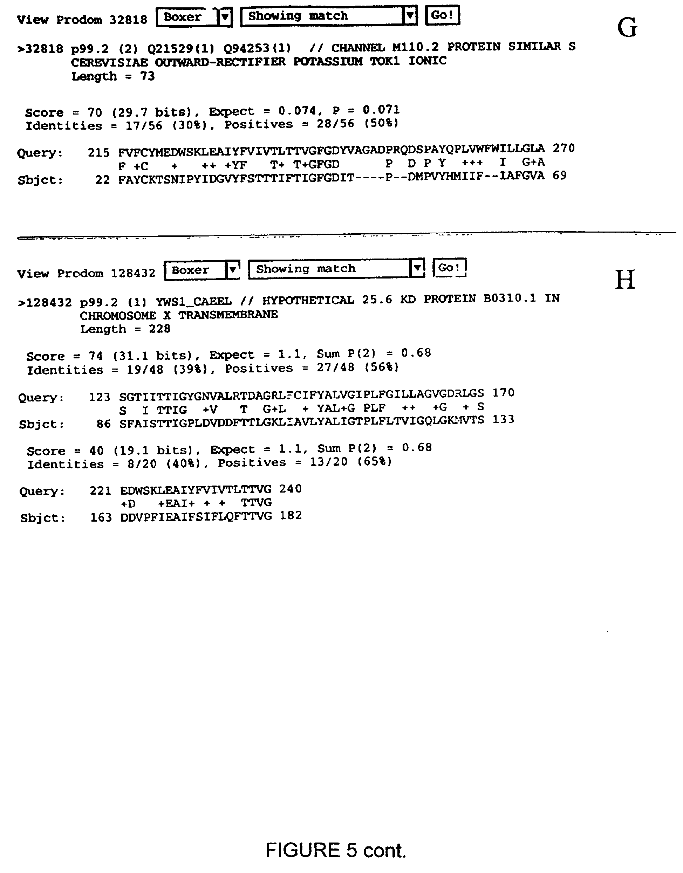

A TWIK-8 molecule can be identified based on the presence of a "TRAAK potassium channel domain" in the protein or corresponding nucleic acid molecule. As used herein, the term "TRAAK potassium channel domain" includes a protein domain having an amino acid sequence of about 20-150 amino acid residues and having a bit score for the alignment of the sequence to the TRAAK potassium channel domain of at least 115-175. Preferably, a TRAAK potassium channel domain includes at least about 23-100, or more preferably about 25, 55, or 95 amino acid residues, and has a bit score for the alignment of the sequence to the TRAAK potassium channel domain of at least 20, 30, 40, 50, 60, 70, 80, 90, 100, 110 or higher. The TRAAK potassium channel domain has been assigned ProDom entries 73512, 98483, and 105542. To identify the presence of a TRAAK potassium channel domain in a TWIK-8 protein, and to make the determination that a protein of interest has a particular profile, the amino acid sequence of the protein is searched against a database of known protein domains (*e.g.*, the ProDom database) using the default parameters (available at http://www.toulouse.inra.fr/prodom.html). A search was performed against the ProDom database resulting in the identification of a TRAAK potassium channel domains in the amino acid sequence of human TWIK-8 (SEQ ID NO:2) at about residues 50-104,175-199, and 288-382 of SEQ ID NO: 2. The results of the search are set forth in Figures 5A, 5B, and 5D.

A TWIK-8 molecule can be identified based on the presence of a "potassium channel protein domain" in the protein or corresponding nucleic acid molecule. As used herein, the term "potassium channel protein domain" includes a protein domain having an amino acid sequence of about 20-100 amino acid residues and having a bit score for the alignment of the sequence to the potassium channel protein domain of at least 101. Preferably, a potassium channel protein domain includes at least about 40-75, or more preferably about 55 amino acid residues, and has a bit score for the alignment of the sequence to the potassium channel protein domain of at least 20, 30, 40, 50, 60, 70, 80, 90, 100, or higher. The potassium channel protein domain has been assigned ProDom entry 129403. To identify the presence of a potassium channel protein domain in a TWIK-8 protein, and to make the determination that a protein of interest has a particular profile, the amino acid sequence of the protein is searched against a database of known protein domains (*e.g.*, the ProDom database) using the default parameters (available at http://www.toulouse.inra.fr/prodom.html). A search was performed against the ProDom database resulting in the identification of a potassium channel protein domain in the amino acid sequence of human TWIK-8 (SEQ ID NO: 2) at about residues 99-153 of SEQ ID NO: 2. The results of the search are set forth in Figure 5F.

A TWIK-8 molecule can be identified based on the presence of a "voltage-gated potassium channel domain" in the protein or corresponding nucleic acid molecule. As used herein, the term "voltage-gated potassium channel domain" includes a protein domain having an amino acid sequence of about 20-100 amino acid residues and having a bit score for the alignment of the sequence to the potassium channel protein domain of at least 115. Preferably, a voltage-gated potassium channel domain includes at least about 40-75, or more preferably about 55 amino acid residues, and has a bit score for the alignment of the sequence to the voltage-gated potassium channel domain of at least 20, 30, 40, 50, 60, 70, 80, 90, 100, 110 or higher. The voltage-gated potassium channel domain has been assigned ProDom entry 36. To identify the presence of a voltage-gated potassium channel domain in a TWIK-8 protein, and to make the determination that a protein of interest has a particular profile, the amino acid sequence of the protein is searched against a database of known protein domains (*e.g.*, the ProDom database) using the default parameters (available at http://www.toulouse.inra.fr/prodom.html). A search was performed against the ProDom database resulting in the identification of a voltage-gated potassium channel domain in the amino acid sequence of human TWIK-8 (SEQ ID NO: 2) at about residues 102-168 of SEQ ID NO: 2. The results of the search are set forth in Figure 5E.

A TWIK-8 molecule can be identified based on the presence of an "outward-rectifier TOK1 potassium channel domain" in the protein or corresponding nucleic acid molecule. As used herein, the term "outward-rectifier TOK1 potassium channel domain" includes a protein domain having an amino acid sequence of about 25-100 amino acid residues and having a bit score for the alignment of the sequence to the outward-rectifier TOK1 potassium channel domain of at least 70. Preferably, an outward-rectifier TOK1 potassium channel domain includes at least about 40-75, or more preferably about 56 amino acid residues, and has a bit score for the alignment of the sequence to the outward-rectifier TOK1 potassium channel domain of at least 20, 30, 40, 50, 60, or higher. The outward-rectifier TOK1 potassium channel domain has been assigned ProDom entry 32818. To identify the presence of an outward-rectifier TOK1 potassium channel domain in a TWIK-8 protein, and to make the determination that a protein of interest has a particular profile, the amino acid sequence of the protein is searched against a database of known protein domains (*e.g.*, the ProDom database) using the default parameters (available at http://www.toulouse.inra.fr/prodom.html). A search was performed against the ProDom database resulting in the identification of an outward-rectifier TOK1 potassium channel domain in the amino acid sequence of human TWIK-8 (SEQ ID NO: 2) at about residues 215-270 of SEQ ID NO: 2. The results of the search are set forth in Figure 5G.

A TWIK-8 molecule can be identified based on the presence of a "potassium channel subunit domain" in the protein or corresponding nucleic acid molecule. As used herein, the term "potassium channel subunit domain" includes a protein domain having an amino acid sequence of about 25-125 amino acid residues and having a bit score for the alignment of the sequence to the potassium channel subunit domain of at least 156. Preferably, a potassium channel subunit domain includes at least about 40-100, or more preferably about 72 amino acid residues, and has a bit score for the alignment of the sequence to the potassium channel subunit domain of at least 20, 30, 40, 50, 60, or higher. The potassium channel subunit domain has been assigned ProDom entry 1641. To identify the presence of a potassium channel subunit domain in a TWIK-8 protein, and to make the determination that a protein of interest has a particular profile, the amino acid sequence of the protein is searched against a database of known protein domains (*e.g.*, the ProDom database) using the default parameters (available at http://www.toulouse.inra.fr/prodom.html). A search was performed against the ProDom database resulting in the identification of a potassium channel subunit domain in the amino acid sequence of human TWIK-8 (SEQ ID NO: 2) at about residues 216-287 of SEQ ID NO: 2. The results of the search are set forth in Figure 5C.

Isolated proteins of the present invention, as defined in the claims, have an amino acid sequence at least 95% identical to the amino acid sequence of SEQ ID NO:2 or are encoded by a nucleotide sequence at least 95% identical to SEQ ID NO: 1 or 3.

As used interchangeably herein, an "TWIK-8 activity", "biological activity of TWIK-8 " or "functional activity of TWIK-8", refers to an activity exerted by a TWIK-8 protein, polypeptide or nucleic acid molecule on a TWIK-8 responsive cell or tissue, or on a TWIK-8 protein substrate, as determined *in vivo,* or *in vitro,* according to standard techniques. In one embodiment, a TWIK-8 activity is a direct activity, such as an association with a TWIK-8-target molecule. As used herein, a "target molecule" or "binding partner" is a molecule with which a TWIK-8 protein binds or interacts in nature, such that TWIK-8-mediated function is achieved. A TWIK-8 target molecule can be a non-TWIK-8 molecule or a TWIK-8 protein or polypeptide of the present invention. In an exemplary embodiment, a TWIK-8 target molecule is a TWIK-8 ligand, *e.g.*, a potassium channel pore-forming subunit or a potassium channel ligand. Alternatively, a TWIK-8 activity is an indirect activity, such as a cellular signaling activity mediated by interaction of the TWIK-8 protein with a TWIK-8 ligand. The biological activities of TWIK-8 are described herein. For example, the TWIK-8 proteins of the present invention can have one or more of the following activities: (1) interacting with a non-TWIK protein molecule; (2) activating a TWIK-dependent signal transduction pathway; (3) modulating the release of neurotransmitters; (4) modulating membrane excitability; (5) influencing the resting potential of membranes, wave forms and frequencies of action potentials, and thresholds of excitation, (6) modulating processes which underlie learning and memory, such as integration of sub-threshold synaptic responses and the conductance of back-propagating action potentials, and (7) mediating nociception.

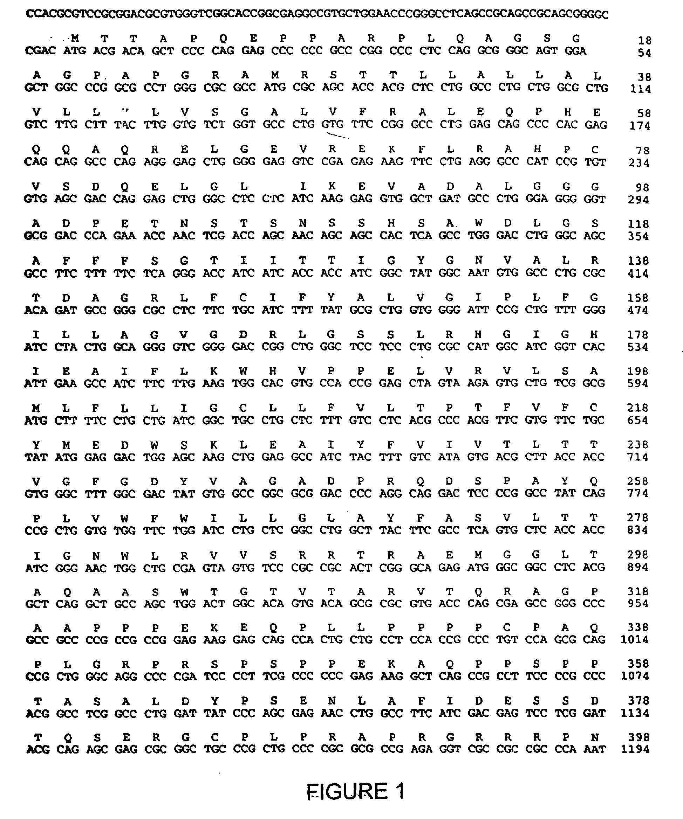

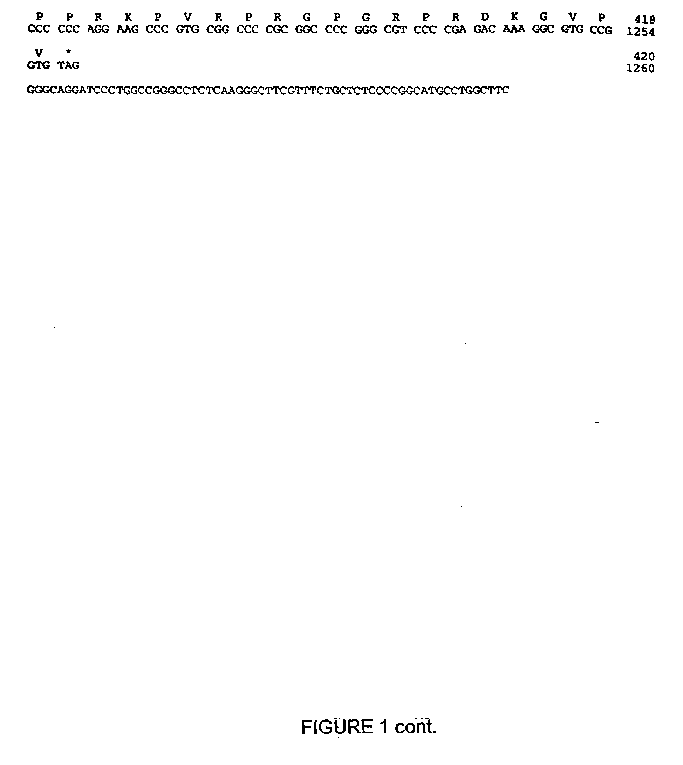

The nucleotide sequence of the isolated human TWIK-8 cDNA and the predicted amino acid sequence of the human TWIK-8 polypeptide are shown in Figure 1 and in SEQ ID NOs:1 and 2, respectively.

The human TWIK-8 gene, which is approximately 1408 nucleotides in length, encodes a protein having a molecular weight of approximately 46.1 kD and which is approximately 419 amino acid residues in length.

Various aspects of the invention are described in further detail in the following subsections:

### I. Isolated Nucleic Acid Molecules

One aspect of the disclosure pertains to isolated nucleic acid molecules that encode TWIK-8 proteins or biologically active portions thereof, as well as nucleic acid fragments sufficient for use as hybridization probes to identify TWIK-8-encoding nucleic acid molecules (*e.g.,* TWIK-8 mRNA) and fragments for use as PCR primers for the amplification or mutation of TWIK-8 nucleic acid molecules. As used herein, the term "nucleic acid molecule" is intended to include DNA molecules (*e.g.*, cDNA or genomic DNA) and RNA molecules (*e.g.*, mRNA) and analogs of the DNA or RNA generated using nucleotide analogs. The nucleic acid molecule can be single-stranded or double-stranded, but preferably is double-stranded DNA.

The term "isolated nucleic acid molecule" includes nucleic acid molecules which are separated from other nucleic acid molecules which are present in the natural source of the nucleic acid. For example, with regards to genomic DNA, the term "isolated" includes nucleic acid molecules which are separated from the chromosome with which the genomic DNA is naturally associated. Preferably, an "isolated" nucleic acid is free of sequences which naturally flank the nucleic acid (*i.e.,* sequences located at the 5' and 3' ends of the nucleic acid) in the genomic DNA of the organism from which the nucleic acid is derived. For example, in various embodiments, the isolated TWIK-8 nucleic acid molecule can contain less than about 5 kb, 4kb, 3kb, 2kb, 1 kb, 0.5 kb or 0.1 kb of nucleotide sequences which naturally flank the nucleic acid molecule in genomic DNA of the cell from which the nucleic acid is derived. Moreover, an "isolated" nucleic acid molecule, such as a cDNA molecule, can be substantially free of other cellular material, or culture medium when produced by recombinant techniques, or substantially free of chemical precursors or other chemicals when chemically synthesized.

A nucleic acid molecule of the present invention, *i.e*, a nucleic acid molecule having the nucleotide sequence of SEQ ID NO:1 or 3, or 95% identity thereof, can be isolated using standard molecular biology techniques and the sequence information provided herein. Using all or portion of the nucleic acid sequence of SEQ ID NO:1 or 3, as a hybridization probe, TWIK-8 nucleic acid molecules can be isolated using standard hybridization and cloning techniques (*e.g.*, as described in Sambrook, J., Fritsh, E. F., and Maniatis, T. *Molecular Cloning: A Laboratory Manual. 2nd ed., Cold Spring Harbor Laboratory,* Cold Spring Harbor Laboratory Press, Cold Spring Harbor, NY, 1989).

Moreover, a nucleic acid molecule encompassing all or a portion of SEQ ID NO:1 or 3, can be isolated by the polymerase chain reaction (PCR) using synthetic oligonucleotide primers designed based upon the sequence of SEQ ID NO:1 or 3.

A nucleic acid of the invention can be amplified using cDNA, mRNA or alternatively, genomic DNA, as a template and appropriate oligonucleotide primers according to standard PCR amplification techniques. The nucleic acid so amplified can be cloned into an appropriate vector and characterized by DNA sequence analysis. Furthermore, oligonucleotides corresponding to TWIK-8 nucleotide sequences can be prepared by standard synthetic techniques, *e.g.*, using an automated DNA synthesizer.

In a preferred embodiment, an isolated nucleic acid molecule of the invention comprises the nucleotide sequence shown in SEQ ID NO:1. The sequence of SEQ ID NO:1 corresponds to the human TWIK-8 cDNA. This cDNA comprises sequences encoding the human TWIK-8 protein (*i.e.,* "the coding region", from nucleotides 84-1343), as well as 5' untranslated sequences (nucleotides 1-83) and 3' untranslated sequences (nucleotides 1344-1408). Alternatively, the nucleic acid molecule can comprise only the coding region of SEQ ID NO:1 (*e.g.,* nucleotides 84-1343, corresponding to SEQ ID NO:3).

In another preferred embodiment, an isolated nucleic acid molecule of the invention comprises a nucleic acid molecule which is a complement of the nucleotide sequence shown in SEQ ID NO:1 or 3. A nucleic acid molecule which is complementary to the nucleotide sequence shown in SEQ ID NO:1 or 3, is one which is sufficiently complementary to the nucleotide sequence shown in SEQ ID NO:1 or 3, such that it can hybridize to the nucleotide sequence shown in SEQ ID NO:1 or 3, thereby forming a stable duplex.

In still another preferred embodiment, an isolated nucleic acid molecule of the present invention comprises a nucleotide sequence which is at least 95%, 96%, 97%, 98%, 99% or more identical to the entire length of the nucleotide sequence shown in SEQ ID NO:1 or 3.

The nucleotide sequence determined from the cloning of the TWIK-8 gene allows for the generation of probes and primers designed for use in identifying and/or cloning other TWIK-8 family members, as well as TWIK-8 homologues from other species. The probe/primer typically comprises substantially purified oligonucleotide. The oligonucleotide typically comprises a region of nucleotide sequence that hybridizes under stringent conditions to at least about 12 or 15, preferably about 20 or 25, more preferably about 30, 35, 40, 45, 50, 55, 60, 65, 75, 80, 85, 90, 95, 100, 150, or 200 or more consecutive nucleotides of a sense sequence of SEQ ID NO:1 or 3, of an anti-sense sequence of SEQ ID NO:1 or 3, or of a naturally occurring allelic variant or mutant of SEQ ID NO:1 or 3.

Probes based on the TWIK-8 nucleotide sequences can be used to detect transcripts or genomic sequences encoding the same or homologous proteins. In preferred embodiments, the probe further comprises a label group attached thereto, *e.g.*, the label group can be a radioisotope, a fluorescent compound, an enzyme, or an enzyme co-factor. Such probes can be used as a part of a diagnostic test kit for identifying cells or tissue which misexpress a TWIK-8 protein, such as by measuring a level of a TWIK-8-encoding nucleic acid in a sample of cells from a subject *e.g.*, detecting TWIK-8 mRNA levels or determining whether a genomic TWIK-8 gene has been mutated or deleted.

A nucleic acid fragment encoding a "biologically active portion of a TWIK-8 protein" can be prepared by isolating a portion of the nucleotide sequence of SEQ ID NO:1 or 3, which encodes a polypeptide having a TWIK-8 biological activity (the biological activities of the TWIK-8 proteins are described herein), expressing the encoded portion of the TWIK-8 protein (*e.g.,* by recombinant expression *in vitro*) and assessing the activity of the encoded portion of the TWIK-8 protein.

The invention further encompasses nucleic acid molecules that differ from the nucleotide sequence shown in SEQ ID NO:1 or 3, due to degeneracy of the genetic code and thus encode the same TWIK-8 proteins as those encoded by the nucleotide sequence shown in SEQ ID NO:1 or 3. In another embodiment, an isolated nucleic acid molecule of the invention has a nucleotide sequence encoding a protein having an amino acid sequence shown in SEQ ID NO:2.

In addition to the TWIK-8 nucleotide sequences shown in SEQ ID NO:1 or 3, it will be appreciated by those skilled in the art that DNA sequence polymorphisms that lead to changes in the amino acid sequences of the TWIK-8 proteins may exist within a population (*e.g.,* the human population). Such genetic polymorphism in the TWIK-8 genes may exist among individuals within a population due to natural allelic variation. As used herein, the terms "gene" and "recombinant gene" refer to nucleic acid molecules which include an open reading frame encoding a TWIK-8 protein, preferably a mammalian TWIK-8 protein, and can further include non-coding regulatory sequences, and introns.

Allelic variants of human TWIK-8 include both functional and non-functional TWIK-8 proteins. Functional allelic variants are naturally occurring amino acid sequence variants of the human TWIK-8 protein that maintain the ability to bind a TWIK-8 ligand or substrate and/or modulate pain signaling mechanisms, membrane excitability or neurotransmitter release. Functional allelic variants will typically contain only conservative substitution of one or more amino acids of SEQ ID NO:2, or substitution, deletion or insertion of non-critical residues in non-critical regions of the protein.

Non-functional allelic variants are naturally occurring amino acid sequence variants of the human TWIK-8 protein that do not have the ability to either bind a TWIK-8 ligand and/or modulate any of the TWIK-8 activities described herein. Non-functional allelic variants will typically contain a non-conservative substitution, a deletion, or insertion or premature truncation of the amino acid sequence of SEQ ID NO:2, or a substitution, insertion or deletion in critical residues or critical regions.

Nucleic acid molecules corresponding to natural allelic variants and homologues of the TWIK-8 cDNAs of the invention can be isolated based on their homology to the TWIK-8 nucleic acids disclosed herein using the cDNAs disclosed herein, or a portion thereof, as a hybridization probe according to standard hybridization techniques under stringent hybridization conditions. Nucleic acid molecules corresponding to natural allelic variants and homologues of the TWIK-8 cDNAs of the invention can further be isolated by mapping to the same chromosome or locus as the TWIK-8 gene.

Accordingly, in another embodiment, an isolated nucleic acid molecule of the invention hybridizes under stringent conditions to the nucleic acid molecule comprising the nucleotide sequence of SEQ ID NO:1 or 3. As used herein, the term "hybridizes under stringent conditions" is intended to describe conditions for hybridization and washing under which nucleotide sequences that are significantly identical or homologous to each other remain hybridized to each other. Preferably, the conditions are such that sequences at least about 70%, more preferably at least about 80%, even more preferably at least about 85% or 90% identical to each other remain hybridized to each other. Such stringent conditions are known to those skilled in the art and can be found in *Current Protocols in Molecular Biology,* Ausubel *et al.,* eds., John Wiley & Sons, Inc. (1995), sections 2, 4 and 6. Additional stringent conditions can be found in *Molecular Cloning: A Laboratory Manual,* Sambrook *et al.,* Cold Spring Harbor Press, Cold Spring Harbor, NY (1989), chapters 7, 9 and 11. A preferred, non-limiting example of stringent hybridization conditions includes hybridization in 4X sodium chloride/sodium citrate (SSC), at about 65-70°C (or hybridization in 4X SSC plus 50% formamide at about 42-50°C) followed by one or more washes in 1X SSC, at about 65-70°C. A preferred, non-limiting example of highly stringent hybridization conditions includes hybridization in 1X SSC, at about 65-70°C (or hybridization in 1X SSC plus 50% formamide at about 42-50°C) followed by one or more washes in 0.3X SSC, at about 65-70°C. A preferred, non-limiting example of reduced stringency hybridization conditions includes hybridization in 4X SSC, at about 50-60°C (or alternatively hybridization in 6X SSC plus 50% formamide at about 40-45°C) followed by one or more washes in 2X SSC, at about 50-60°C. Ranges intermediate to the above-recited values, *e.g.*, at 65-70°C or at 42-50°C are also intended to be encompassed by the present invention. SSPE (1xSSPE is 0.15M NaCl, 10mM NaH₂PO₄, and 1.25mM EDTA, pH 7.4) can be substituted for SSC (1xSSC is 0.15M NaCl and 15mM sodium citrate) in the hybridization and wash buffers; washes are performed for 15 minutes each after hybridization is complete. The hybridization temperature for hybrids anticipated to be less than 50 base pairs in length should be 5-10°C less than the melting temperature (Tₘ) of the hybrid, where Tₘ is determined according to the following equations. For hybrids less than 18 base pairs in length, Tₘ(°C) = 2(# of A + T bases) + 4(# of G + C bases). For hybrids between 18 and 49 base pairs in length, Tₘ(°C) = 81.5 + 16.6(log₁₀[Na⁺]) + 0.41(%G+C)- (600/N), where N is the number of bases in the hybrid, and [Na⁺] is the concentration of sodium ions in the hybridization buffer ([Na⁺] for 1xSSC = 0.165 M). It will also be recognized by the skilled practitioner that additional reagents may be added to hybridization and/or wash buffers to decrease non-specific hybridization of nucleic acid molecules to membranes, for example, nitrocellulose or nylon membranes, including but not limited to blocking agents (*e.g.*, BSA or salmon or herring sperm carrier DNA), detergents (*e.g.*, SDS), chelating agents (*e.g.*, EDTA), Ficoll, PVP and the like. When using nylon membranes, in particular, an additional preferred, non-limiting example of stringent hybridization conditions is hybridization in 0.25-0.5M NaH₂PO₄, 7% SDS at about 65°C, followed by one or more washes at 0.02M NaH₂PO₄, 1% SDS at 65°C, see *e.g.*, Church and Gilbert (1984) *Proc. Natl. Acad. Sci. USA* 81:1991-1995, (or alternatively 0.2X SSC, 1% SDS).

Preferably, an isolated nucleic acid molecule hybridizes under stringent conditions to the sequence of SEQ ID NO:1 or 3 and corresponds to a naturally-occurring nucleic acid molecule. As used herein, a "naturally-occurring" nucleic acid molecule refers to an RNA or DNA molecule having a nucleotide sequence that occurs in nature (*e.g.*, encodes a natural protein).

In addition to naturally-occurring allelic variants of the TWIK-8 sequences that may exist in the population, the skilled artisan will further appreciate that changes can be introduced by mutation into the nucleotide sequences of SEQ ID NO:1 or 3, thereby leading to changes in the amino acid sequence of the encoded TWIK.-8 proteins, without altering the functional ability of the TWIK-8 proteins. For example, nucleotide substitutions leading to amino acid substitutions at "non-essential" amino acid residues can be made in the sequence of SEQ ID NO:1 or 3. A "non-essential" amino acid residue is a residue that can be altered from the wild-type sequence of TWIK-8 (*e.g.,* the sequence of SEQ ID NO:2) without altering the biological activity, whereas an "essential" amino acid residue is required for biological activity. For example, amino acid residues that are conserved among the TWIK-8 proteins of the present invention, *e.g.*, those present in a transmembrane domain, are predicted to be particularly unamenable to alteration. Furthermore, additional amino acid residues that are conserved between the TWIK-8 proteins of the present invention and other members of the TWIK family are not likely to be amenable to alteration.

Accordingly, another aspect of the invention pertains to nucleic acid molecules encoding TWIK-8 proteins that contain changes in amino acid residues that are not essential for activity. Such TWIK-8 proteins differ in amino acid sequence from SEQ ID NO:2, yet retain biological activity. In one embodiment, the isolated nucleic acid molecule comprises a nucleotide sequence encoding a protein, wherein the protein comprises an amino acid sequence at least 95%, 96%, 97%, 98%, 99% or more identical to SEQ ID NO:2.

An isolated nucleic acid molecule encoding a TWIK-8 protein identical to the protein of SEQ ID NO:2, can be created by introducing one or more nucleotide substitutions, additions or deletions into the nucleotide sequence of SEQ ID NO:1 or 3, such that one or more amino acid substitutions, additions or deletions are introduced into the encoded protein. Mutations can be introduced into SEQ ID NO:1 or 3, by standard techniques, such as site-directed mutagenesis and PCR-mediated mutagenesis. Preferably, conservative amino acid substitutions are made at one or more predicted non-essential amino acid residues. A "conservative amino acid substitution" is one in which the amino acid residue is replaced with an amino acid residue having a similar side chain. Families of amino acid residues having similar side chains have been defined in the art. These families include amino acids with basic side chains (*e.g.*, lysine, arginine, histidine), acidic side chains (*e.g.*, aspartic acid, glutamic acid), uncharged polar side chains (*e.g.*, glycine, asparagine, glutamine, serine, threonine, tyrosine, cysteine), nonpolar side chains (*e.g.*, alanine, valine, leucine, isoleucine, proline, phenylalanine, methionine, tryptophan), beta-branched side chains (*e.g.,* threonine, valine, isoleucine) and aromatic side chains (*e.g.*, tyrosine, phenylalanine, tryptophan, histidine). Thus, a predicted nonessential amino acid residue in a TWIK-8 protein is preferably replaced with another amino acid residue from the same side chain family. Alternatively, in another embodiment, mutations can be introduced randomly along all or part of a TWIK-8 coding sequence, such as by saturation mutagenesis, and the resultant mutants can be screened for TWIK-8 biological activity to identify mutants that retain activity. Following mutagenesis of SEQ ID NO:1 or 3, the encoded protein can be expressed recombinantly and the activity of the protein can be determined.

In a preferred embodiment, a mutant TWIK-8 protein can be assayed for the ability to (1) interact with a non-TWIK protein molecule; (2) activate a TWIK-dependent signal transduction pathway; (3) modulate the release of neurotransmitters; (4) modulate membrane excitability; (5) influence the resting potential of membranes, wave forms and frequencies of action potentials, and thresholds of excitation, (6) modulate processes which underlie learning and memory, such as integration of sub-threshold synaptic responses and the conductance of back-propagating action potentials, and (7) mediate nociception.

In addition to the nucleic acid molecules encoding TWIK-8 proteins described above, another aspect of the invention pertains to isolated nucleic acid molecules which are antisense thereto. An "antisense" nucleic acid comprises a nucleotide sequence which is complementary to a "sense" nucleic acid encoding a protein, *e.g.*, complementary to the coding strand of a double-stranded cDNA molecule or complementary to an mRNA sequence. Accordingly, an antisense nucleic acid can hydrogen bond to a sense nucleic acid. The antisense nucleic acid can be complementary to an entire TWIK-8 coding strand, or to only a portion thereof. In one embodiment, an antisense nucleic acid molecule is antisense to a "coding region" of the coding strand of a nucleotide sequence encoding TWIK-8. The term "coding region" refers to the region of the nucleotide sequence comprising codons which are translated into amino acid residues (*e.g.*, the coding region of human TWIK-8 corresponds to SEQ ID NO:3). In another embodiment, the antisense nucleic acid molecule is antisense to a "noncoding region" of the coding strand of a nucleotide sequence encoding TWIK-8. The term "noncoding region" refers to 5' and 3' sequences which flank the coding region that are not translated into amino acids (*i.e.,* also referred to as 5' and 3' untranslated regions).

Given the coding strand sequences encoding TWIK-8 disclosed herein (*e.g.,* SEQ ID NO:3), antisense nucleic acids of the invention can be designed according to the rules of Watson and Crick base pairing. The antisense nucleic acid molecule can be complementary to the entire coding region of TWIK-8 mRNA, but more preferably is an oligonucleotide which is antisense to only a portion of the coding or noncoding region of TWIK-8 mRNA. For example, the antisense oligonucleotide can be complementary to the region surrounding the translation start site of TWIK-8 mRNA. An antisense oligonucleotide can be, for example, about 5, 10, 15, 20, 25, 30, 35, 40, 45 or 50 nucleotides in length. These fragments are not part of the invention. An antisense nucleic acid can be constructed using chemical synthesis and enzymatic ligation reactions using procedures known in the art. For example, an antisense nucleic acid (*e.g.*, an antisense oligonucleotide) can be chemically synthesized using naturally occurring nucleotides or variously modified nucleotides designed to increase the biological stability of the molecules or to increase the physical stability of the duplex formed between the antisense and sense nucleic acids, *e.g.*, phosphorothioate derivatives and acridine substituted nucleotides can be used. Examples of modified nucleotides which can be used to generate the antisense nucleic acid include 5-fluorouracil, 5-bromouracil, 5-chlorouracil, 5-iodouracil, hypoxanthine, xantine, 4-acetylcytosine, 5-(carboxyhydroxylmethyl) uracil, 5-carboxymethylaminomethyl-2-thiouridine, 5-carboxymethylaminomethyluracil, dihydrouracil, beta-D-galactosylqueosine, inosine, N6-isopentenyladenine, 1-methylguanine, 1-methylinosine, 2,2-dimethylguanine, 2-methyladenine, 2-methylguanine, 3-methylcytosine, 5-methylcytosine, N6-adenine, 7-methylguanine, 5-methylaminomethyluracil, 5-methoxyaminomethyl-2-thiouracil, beta-D-mannosylqueosine, 5'-methoxycarboxymethyluracil, 5-methoxyuracil, 2-methylthio-N6-isopentenyladenine, uracil-5-oxyacetic acid (v), wybutoxosine, pseudouracil, queosine, 2-thiocytosine, 5-methyl-2-thiouracil, 2-thiouracil, 4-thiouracil, 5-methyluracil, uracil-5-oxyacetic acid methylester, uracil-5-oxyacetic acid (v), 5-methyl-2-thiouracil, 3-(3-amino-3-N-2-carboxypropyl) uracil, (acp3)w, and 2,6-diaminopurine. Alternatively, the antisense nucleic acid can be produced biologically using an expression vector into which a nucleic acid has been subcloned in an antisense orientation (*i.e.,* RNA transcribed from the inserted nucleic acid will be of an antisense orientation to a target nucleic acid of interest, described further in the following subsection).

The antisense nucleic acid molecules are typically administered to a subject or generated *in situ* such that they hybridize with or bind to cellular mRNA and/or genomic DNA encoding a TWIK-8 protein to thereby inhibit expression of the protein, *e.g.*, by inhibiting transcription and/or translation. The hybridization can be by conventional nucleotide complementarity to form a stable duplex, or, for example, in the case of an antisense nucleic acid molecule which binds to DNA duplexes, through specific interactions in the major groove of the double helix. An example of a route of administration of antisense nucleic acid molecules of the invention include direct injection at a tissue site. Alternatively, antisense nucleic acid molecules can be modified to target selected cells and then administered systemically. For example, for systemic administration, antisense molecules can be modified such that they specifically bind to receptors or antigens expressed on a selected cell surface, *e.g.*, by linking the antisense nucleic acid molecules to peptides or antibodies which bind to cell surface receptors or antigens. The antisense nucleic acid molecules can also be delivered to cells using the vectors described herein. To achieve sufficient intracellular concentrations of the antisense molecules, vector constructs in which the antisense nucleic acid molecule is placed under the control of a strong pol II or pol III promoter are preferred.

In yet another embodiment, the antisense nucleic acid molecule is an a-anomeric nucleic acid molecule. An α-anomeric nucleic acid molecule forms specific double-stranded hybrids with complementary RNA in which, contrary to the usual β-units, the strands run parallel to each other (Gaultier *et al.* (1987) *Nucleic Acids. Res.* 15:6625-6641). The antisense nucleic acid molecule can also comprise a 2'-o-methylribonucleotide (Inoue *et al.* (1987) *Nucleic Acids Res.* 15:6131-6148) or a chimeric RNA-DNA analogue (Inoue *et al.* (1987) *FEBS Lett.* 215:327-330).

In still another embodiment, an antisense nucleic acid is a ribozyme. Ribozymes are catalytic RNA molecules with ribonuclease activity which are capable of cleaving a single-stranded nucleic acid, such as an mRNA, to which they have a complementary region. Thus, ribozymes (*e.g.*, hammerhead ribozymes (described in Haselhoff and Gerlach (1988) *Nature* 334:585-591)) can be used to catalytically cleave TWIK-8 mRNA transcripts to thereby inhibit translation of TWIK-8 mRNA. A ribozyme having specificity for a TWIK-8-encoding nucleic acid can be designed based upon the nucleotide sequence of a TWIK-8 cDNA disclosed herein (*i.e.,* SEQ ID NO:1 or 3). For example, a derivative of a *Tetrahymena* L-19 IVS RNA can be constructed in which the nucleotide sequence of the active site is complementary to the nucleotide sequence to be cleaved in a TWIK-8-encoding mRNA. See, *e.g.*, Cech *et al.* U.S. Patent No. 4,987,071; and Cech *et al.* U.S. Patent No. 5,116,742. Alternatively, TWIK-8 mRNA can be used to select a catalytic RNA having a specific ribonuclease activity from a pool of RNA molecules. See, *e.g.*, Bartel, D. and Szostak, J.W. (1993) *Science* 261:1411-1418.

Alternatively, the expression characteristics of an endogenous TWIK-8 gene within a cell line or microorganism may be modified by inserting a heterologous DNA regulatory element into the genome of a stable cell line or cloned microorganism such that the inserted regulatory element is operatively linked with the endogenous TWIK-8 gene. For example, an endogenous TWIK-8 gene which is normally "transcriptionally silent", *i.e.,* a TWIK-8 gene which is normally not expressed, or is expressed only at very low levels in a cell line or microorganism, may be activated by inserting a regulatory element which is capable of promoting the expression of a normally expressed gene product in that cell line or microorganism. Alternatively, a transcriptionally silent, endogenous TWIK-8 gene may be activated by insertion of a promiscuous regulatory element that works across cell types.

A heterologous regulatory element may be inserted into a stable cell line or cloned microorganism, such that it is operatively linked with an endogenous TWIK-8 gene, using techniques, such as targeted homologous recombination, which are well known to those of skill in the art, and described, *e.g.*, in Chappel, U.S. Patent No. 5,272,071; PCT publication No. WO 91/06667, published May 16, 1991.

### II. Isolated TWIK-8 Proteins and Anti-TWIK-8 Antibodies

One aspect of the invention pertains to isolated TWIK-8 proteins, for use as immunogens to raise anti-TWIK-8 antibodies. In one embodiment, native TWIK-8 proteins can be isolated from cells or tissue sources by an appropriate purification scheme using standard protein purification techniques. In another embodiment, TWIK-8 proteins are produced by recombinant DNA techniques. Alternative to recombinant expression, a TWIK-8 protein can be synthesized chemically using standard peptide synthesis techniques.

An "isolated" or "purified" protein is substantially free of cellular material or other contaminating proteins from the cell or tissue source from which the TWIK-8 protein is derived, or substantially free from chemical precursors or other chemicals when chemically synthesized. The language "substantially free of cellular material" includes preparations of TWIK-8 protein in which the protein is separated from cellular components of the cells from which it is isolated or recombinantly produced. In one embodiment, the language "substantially free of cellular material" includes preparations of TWIK-8 protein having less than about 30% (by dry weight) of non-TWIK-8 protein (also referred to herein as a "contaminating protein"), more preferably less than about 20% of non-TWIK-8 protein, still more preferably less than about 10% of non-TWIK-8 protein, and most preferably less than about 5% non-TWIK-8 protein. When the TWIK-8 protein is recombinantly produced, it is also preferably substantially free of culture medium, *i.e.,* culture medium represents less than about 20%, more preferably less than about 10%, and most preferably less than about 5% of the volume of the protein preparation.

The language "substantially free of chemical precursors or other chemicals" includes preparations of TWIK-8 protein in which the protein is separated from chemical precursors or other chemicals which are involved in the synthesis of the protein. In one embodiment, the language "substantially free of chemical precursors or other chemicals" includes preparations of TWIK-8 protein having less than about 30% (by dry weight) of chemical precursors or non-TWIK-8 chemicals, more preferably less than about 20% chemical precursors or non-TWIK-8 chemicals, still more preferably less than about 10% chemical precursors or non-T'WIK-8 chemicals, and most preferably less than about 5% chemical precursors or non-TWIK-8 chemicals.

In a preferred embodiment, the TWIK-8 protein has an amino acid sequence shown in SEQ ID NO:2. In other embodiments, the TWIK.-8 protein is substantially identical to SEQ ID NO:2, and retains the functional activity of the protein of SEQ ID NO:2, yet differs in amino acid sequence due to natural allelic variation or mutagenesis, as described in detail in subsection I above. Accordingly, in another embodiment, the TWIK-8 protein is a protein which comprises an amino acid sequence at least about 95%, 96%, 97%, 98%, 99% or more identical to SEQ ID NO:2.

To determine the percent identity of two amino acid sequences or of two nucleic acid sequences, the sequences are aligned for optimal comparison purposes (*e.g.*, gaps can be introduced in one or both of a first and a second amino acid or nucleic acid sequence for optimal alignment and non-identical sequences can be disregarded for comparison purposes). The amino acid residues or nucleotides at corresponding amino acid positions or nucleotide positions are then compared. When a position in the first sequence is occupied by the same amino acid residue or nucleotide as the corresponding position in the second sequence, then the molecules are identical at that position (as used herein amino acid or nucleic acid "identity" is equivalent to amino acid or nucleic acid "homology"). The percent identity between the two sequences is a function of the number of identical positions shared by the sequences, taking into account the number of gaps, and the length of each gap, which need to be introduced for optimal alignment of the two sequences.

The comparison of sequences and determination of percent identity between two sequences can be accomplished using a mathematical algorithm. In a preferred embodiment, the percent identity between two amino acid sequences is determined using the Needleman and Wunsch (*J. Mol. Biol.* (48):444-453 (1970)) algorithm which has been incorporated into the GAP program in the GCG software package (available at http://www.gcg.com), using either a Blosum 62 matrix or a PAM250 matrix, and a gap weight of 16, 14, 12, 10, 8, 6, or 4 and a length weight of 1, 2, 3, 4, 5, or 6. In yet another preferred embodiment, the percent identity between two nucleotide sequences is determined using the GAP program in the GCG software package (available at http://www.gcg.com), using a NWSgapdna.CMP matrix and a gap weight of 40, 50,60, 70, or 80 and a length weight of 1, 2, 3, 4, 5, or 6. In another embodiment, the percent identity between two amino acid or nucleotide sequences is determined using the algorithm of E. Meyers and W. Miller (CABIOS, 4:11-17 (1989)) which has been incorporated into the ALIGN program (version 2.0), using a PAM120 weight residue table, a gap length penalty of 12 and a gap penalty of 4.

The nucleic acid and protein sequences of the present invention can further be used as a "query sequence" to perform a search against public databases to, for example, identify other family members or related sequences. Such searches can be performed using the NBLAST and XBLAST programs (version 2.0) of Altschul, *et al.* (1990) *J. Mol. Biol.* 215:403-10. BLAST nucleotide searches can be performed with the NBLAST program, score = 100, wordlength =12 to obtain nucleotide sequences homologous to TWIK-8 nucleic acid molecules of the invention. BLAST protein searches can be performed with the XBLAST program, score =100, wordlength = 3 to obtain amino acid sequences homologous to TWIK-8 protein molecules of the invention. To obtain gapped alignments for comparison purposes, Gapped BLAST can be utilized as described in Altschul *et al.,* (1997) *Nucleic Acids Res.* 25(17):3389-3402. When utilizing BLAST and Gapped BLAST programs, the default parameters of the respective programs (*e.g.*, XBLAST and NBLAST) can be used. See http://www.ncbi.nlm.nih.gov.

The invention also provides TWIK-8 chimeric or fusion proteins. As used herein, a TWIK-8 "chimeric protein" or "fusion protein" comprises a TWIK-8 polypeptide operatively linked to a non-TWIK-8 polypeptide. An "TWIK-8 polypeptide" refers to a polypeptide having an amino acid sequence corresponding to TWIK-8, whereas a "non-TWIK-8 polypeptide" refers to a polypeptide having an amino acid sequence corresponding to a protein which is not substantially homologous to the TWIK-8 protein, *e.g.*, a protein which is different from the TWIK-8 protein and which is derived from the same or a different organism. Within the fusion protein, the term "operatively linked" is intended to indicate that the TWIK-8 polypeptide and the non-TWIK-8 polypeptide are fused in-fiame to each other. The non-TWIK-8 polypeptide can be fused to the N-terminus or C-terminus of the TWIK-8 polypeptide.

For example, in one embodiment, the fusion protein is a GST-TWIK-8 fusion protein in which the TWIK-8 sequences are fused to the C-terminus of the GST sequences. Such fusion proteins can facilitate the purification of recombinant TWIK-8.

In another embodiment, the fusion protein is a TWIK-8 protein containing a heterologous signal sequence at its N-terminus. In certain host cells (*e.g.*, mammalian host cells), expression and/or secretion of TWIK-8 can be increased through use of a heterologous signal sequence.

Preferably, a TWIK-8 chimeric or fusion protein of the invention is produced by standard recombinant DNA techniques. For example, DNA fragments coding for the different polypeptide sequences are ligated together in-frame in accordance with conventional techniques, for example by employing blunt-ended or stagger-ended termini for ligation, restriction enzyme digestion to provide for appropriate termini, filling-in of cohesive ends as appropriate, alkaline phosphatase treatment to avoid undesirable joining, and enzymatic ligation. In another embodiment, the fusion gene can be synthesized by conventional techniques including automated DNA synthesizers. Alternatively, PCR amplification of gene fragments can be carried out using anchor primers which give rise to complementary overhangs between two consecutive gene fragments which can subsequently be annealed and reamplified to generate a chimeric gene sequence (see, for example, *Current Protocols in Molecular Biology,* eds. Ausubel *et al.* John Wiley & Sons: 1992). Moreover, many expression vectors are commercially available that already encode a fusion moiety (*e.g.*, a GST polypeptide). A TWIK-8-encoding nucleic acid can be cloned into such an expression vector such that the fusion moiety is linked in-frame to the TWIK-8 protein.

An isolated TWIK-8 protein, or a portion or fragment thereof, can be used as an immunogen to generate antibodies that bind TWIK-8 using standard techniques for polyclonal and monoclonal antibody preparation. A full-length TWIK-8 protein or, alternatively, antigenic peptide fragments of TWIK-8 can be used as immunogens. The antigenic peptide of TWIK-8 comprises at least 8 amino acid residues of the amino acid sequence shown in SEQ ID NO:2 and encompasses an epitope of TWIK-8 such that an antibody raised against the peptide forms a specific immune complex with TWIK-8. Preferably, the antigenic peptide comprises at least 10 amino acid residues, more preferably at least 15 amino acid residues, even more preferably at least 20 amino acid residues, and most preferably at least 30 amino acid residues.

Preferred epitopes encompassed by the antigenic peptide are regions of TWIK-8 that are located on the surface of the protein, *e.g.*, hydrophilic regions, as well as regions with high antigenicity.

A TWIK-8 immunogen typically is used to prepare antibodies by immunizing a suitable subject, (*e.g.*, rabbit, goat, mouse or other mammal) with the immunogen. An appropriate immunogenic preparation can contain, for example, recombinantly expressed TWIK-8 protein or a chemically synthesized TWIK-8 polypeptide. The preparation can further include an adjuvant, such as Freund's complete or incomplete adjuvant, or similar immunostimulatory agent. Immunization of a suitable subject with an immunogenic TWIK-8 preparation induces a polyclonal anti-TWIK-8 antibody response.

Accordingly, another aspect pertains to anti-TWIK-8 antibodies. The term "antibody" as used herein refers to immunoglobulin molecules and immunologically active portions of immunoglobulin molecules, *i.e.,* molecules that contain an antigen binding site which specifically binds (immunoreacts with) an antigen, such as TWIK-8. Examples of immunologically active portions of immunoglobulin molecules include F(ab) and F(ab')₂ fragments which can be generated by treating the antibody with an enzyme such as pepsin. The invention provides polyclonal and monoclonal antibodies that specifically bind TWIK.-8. The term "monoclonal antibody" or "monoclonal antibody composition", as used herein, refers to a population of antibody molecules that contain only one species of an antigen binding site capable of immunoreacting with a particular epitope of TWIK-8. A monoclonal antibody composition thus typically displays a single binding affinity for a particular TWIK-8 protein with which it immunoreacts.

Polyclonal anti-TWIK-8 antibodies can be prepared as described above by immunizing a suitable subject with a TWIK-8 immunogen. The anti-TWIK-8 antibody titer in the immunized subject can be monitored over time by standard techniques, such as with an enzyme linked immunosorbent assay (ELISA) using immobilized TWIK-8. If desired, the antibody molecules directed against TWIK-8 can be isolated from the mammal (*e.g.*, from the blood) and further purified by well known techniques, such as protein A chromatography to obtain the IgG fraction. At an appropriate time after immunization, *e.g.,* when the anti-TWIK-8 antibody titers are highest, antibody-producing cells can be obtained from the subject and used to prepare monoclonal antibodies by standard techniques, such as the hybridoma technique originally described by Kohler and Milstein (1975) *Nature* 256:495-497) (see also, Brown *et al.* (1981) *J. Immunol.* 127:539-46; Brown *et al.* (1980) *J. Biol. Chem* .255:4980-83; Yeh *et al.* (1976) *Proc. Natl. Acad Sci. USA* 76:2927-31; and Yeh *et al.* (1982) *Int. J. Cancer* 29:269-75), the more recent human B cell hybridoma technique (Kozbor *et al.* (1983) *Immunol Today* 4:72), the EBV-hybridoma technique (Cole *et al.* (1985), *Monoclonal Antibodies and Cancer Therapy,* Alan R. Liss, Inc., pp. 77-96) or trioma techniques. The technology for producing monoclonal antibody hybridomas is well known (see generally R. H. Kenneth, in *Monoclonal Antibodies: A New Dimension In Biological Analyses,* Plenum Publishing Corp., New York, New York (1980); E. A. Lerner (1981) *Yale J. Biol. Med ,* 54:387-402; M. L. Gefter *et al.* (1977) *Somatic Cell Genet.* 3:231-36). Briefly, an immortal cell line (typically a myeloma) is fused to lymphocytes (typically splenocytes) from a mammal immunized with a TWIK-8 immunogen as described above, and the culture supernatants of the resulting hybridoma cells are screened to identify a hybridoma producing a monoclonal antibody that binds TWIK-8.

Any of the many well known protocols used for fusing lymphocytes and immortalized cell lines can be applied for the purpose of generating an anti-TWIK-8 monoclonal antibody (see, *e.g.,* G. Galfre *et al.* (1977) *Nature* 266:55052; Gefter *et al. Somatic Cell Genet.,* cited *supra;* Lerner, *Yale J. Biol. Med,* cited *supra;* Kenneth, *Monoclonal Antibodies,* cited *supra).* Moreover, the ordinarily skilled worker will appreciate that there are many variations of such methods which also would be useful. Typically, the immortal cell line (*e.g.*, a myeloma cell line) is derived from the same mammalian species as the lymphocytes. For example, murine hybridomas can be made by fusing lymphocytes from a mouse immunized with an immunogenic preparation of the present invention with an immortalized mouse cell line. Preferred immortal cell lines are mouse myeloma cell lines that are sensitive to culture medium containing hypoxanthine, aminopterin and thymidine ("HAT medium"). Any of a number of myeloma cell lines can be used as a fusion partner according to standard techniques, *e.g.,* the P3-NS1/1-Ag4-1, P3-x63-Ag8.653 or Sp2/O-Ag14 myeloma lines. These myeloma lines are available from ATCC. Typically, HAT-sensitive mouse myeloma cells are fused to mouse splenocytes using polyethylene glycol ("PEG"). Hybridoma cells resulting from the fusion are then selected using HAT medium, which kills unfused and unproductively fused myeloma cells (unfused splenocytes die after several days because they are not transformed). Hybridoma cells producing a monoclonal antibody of the invention are detected by screening the hybridoma culture supernatants for antibodies that bind TWIK-8, *e.g.*, using a standard ELISA assay.

Alternative to preparing monoclonal antibody-secreting hybridomas, a monoclonal anti-TWIK-8 antibody can be identified and isolated by screening a recombinant combinatorial immunoglobulin library (*e.g.*, an antibody phage display library) with TWIK-8 to thereby isolate immunoglobulin library members that bind TWIK-8. Kits for generating and screening phage display libraries are commercially available (*e.g.,* the Pharmacia *Recombinant Phage Antibody System,* Catalog No. 27-9400-01; and the Stratagene *SurfZAP*^{™} *Phage Display Kit,* Catalog No. 240612). Additionally, examples of methods and reagents particularly amenable for use in generating and screening antibody display library can be found in, for example, Ladner *et al.* U.S. Patent No. 5,223,409; Kang *et al.* PCT International Publication No. WO 92/18619; Dower *et al.* PCT International Publication No. WO 91/17271; Winter *et al.* PCT International Publication WO 92/20791; Markland *et al.* PCT International Publication No. WO 92/15679; Breitling *et al.* PCT International Publication WO 93/01288; McCafferty *et al.* PCT International Publication No. WO 92/01047; Garrard *et al.* PCT International Publication No. WO 92/09690; Ladner *et al.* PCT International Publication No. WO 90/02809; Fuchs *et al.* (1991) *BiolTechnology* 9:1370-1372; Hay *et al.* (1992) *Hum. Antibod. Hybridomas* 3:81-85; Huse *et al.* (1989) *Science* 246:1275-1281; Griffiths *et al.* (1993) *EMBO J* 12:725-734; Hawkins *et al.* (1992) *J. Mol. Biol.* 226:889-896; Clarkson *et al.* (1991) *Nature* 352:624-628; Gram *et al.* (1992) *Proc. Natl. Acad. Sci. USA* 89:3576-3580; Garrad *et al.* (1991) *Bio*/*Technology* 9:1373-1377; Hoogenboom *et al.* (1991) *Nuc. Acid Res.* 19:4133-4137; Barbas *et al.* (1991) *Proc. Natl. Acad. Sci. USA* 88:7978-7982; and McCafferty *et al. Nature* (1990) 348:552-554.

Additionally, recombinant anti-TWIK-8 antibodies, such as chimeric and humanized monoclonal antibodies, comprising both human and non-human portions, which can be made using standard recombinant DNA techniques, are within the scope of the invention. Such chimeric and humanized monoclonal antibodies can be produced by recombinant DNA techniques known in the art, for example using methods described in Robinson *et al.* International Application No. PCT/LTS86/02269; Akira, *et al.* European Patent Application 184,187; Taniguchi, M., European Patent Application 171,496; Morrison *et al.* European Patent Application 173,494; Neuberger *et al.* PCT International Publication No. WO 86/01533; Cabilly *et al.* U.S. Patent No. 4,816,567; Cabilly *et al.* European Patent Application 125,023; Better *et al.* (1988) *Science* 240:1041-1043; *Liu et al.* (1987) *Proc. Natl. Acad. Sci. USA* 84:3439-3443; Liu *et al.* (1987) *J. Immunol.* 139:3521-3526; Sun *et al. (1987) Proc. Natl. Acad. Sci. USA* 84:214-218; Nishimura *et al.* (1987) *Canc. Res.* 47:999-1005; Wood *et al.* (1985) *Nature* 314:446-449; and Shaw *et al.* (1988) *J. Natl. Cancer Inst.* 80:1553-1559); Morrison, S. L. (1985) *Science* 229:1202-1207; Oi *et al.* (1986) *BioTechniques* 4*:214;* Winter U.S. Patent 5,225,539; Jones *et al.* (1986) *Nature* 321:552-525; Verhoeyan *et al.* (1988) *Science* 239:1534; and Beidler *et al.* (1988) *J. Immunol.* 141:4053-4060.

An anti-TWIK-8 antibody (*e.g.,* monoclonal antibody) can be used to isolate TWIK-8 by standard techniques, such as affinity chromatography or immunoprecipitation. An anti-TWIK-8 antibody can facilitate the purification of natural TWIK-8 from cells and of recombinantly produced TWIK-8 expressed in host cells. Moreover, an anti-TWIK-8 antibody can be used to detect TWIK-8 protein (*e.g.*, in a cellular lysate or cell supernatant) in order to evaluate the abundance and pattern of expression of the TWIK-8 protein. Anti-TWIK-8 antibodies can be used diagnostically to monitor protein levels in tissue as part of a clinical testing procedure, *e.g.*, to, for example, determine the efficacy of a given treatment regimen. Detection can be facilitated by coupling (i. e., physically linking) the antibody to a detectable substance. Examples of detectable substances include various enzymes, prosthetic groups, fluorescent materials, luminescent materials, bioluminescent materials, and radioactive materials. Examples of suitable enzymes include horseradish peroxidase, alkaline phosphatase, β-galactosidase, or acetylcholinesterase; examples of suitable prosthetic group complexes include streptavidin/biotin and avidin/biotin; examples of suitable fluorescent materials include umbelliferone, fluorescein, fluorescein isothiocyanate, rhodamine, dichlorotriazinylamine fluorescein, dansyl chloride or phycoerythrin; an example of a luminescent material includes luminol; examples of bioluminescent materials include luciferase, luciferin, and aequorin, and examples of suitable radioactive material include ¹²⁵I, ¹³¹I, ³⁵S or ³H.

### III. Recombinant Expression Vectors and Host Cells

Another aspect of the invention pertains to vectors, preferably expression vectors, containing a nucleic acid encoding a TWIK-8 protein. As used herein, the term "vector" refers to a nucleic acid molecule capable of transporting another nucleic acid to which it has been linked. One type of vector is a "plasmid", which refers to a circular double stranded DNA loop into which additional DNA segments can be ligated. Another type of vector is a viral vector, wherein additional DNA segments can be ligated into the viral genome. Certain vectors are capable of autonomous replication in a host cell into which they are introduced (*e.g.*, bacterial vectors having a bacterial origin of replication and episomal mammalian vectors). Other vectors (*e.g.*, non-episomal mammalian vectors) are integrated into the genome of a host cell upon introduction into the host cell, and thereby are replicated along with the host genome. Moreover, certain vectors are capable of directing the expression of genes to which they are operatively linked. Such vectors are referred to herein as "expression vectors". In general, expression vectors of utility in recombinant DNA techniques are often in the form of plasmids. In the present specification, "plasmid" and "vector" can be used interchangeably as the plasmid is the most commonly used form of vector. However, the invention is intended to include such other forms of expression vectors, such as viral vectors (*e.g.,* replication defective retroviruses, adenoviruses and adeno-associated viruses), which serve equivalent functions.

The recombinant expression vectors of the invention comprise a nucleic acid of the invention in a form suitable for expression of the nucleic acid in a host cell, which means that the recombinant expression vectors include one or more regulatory sequences, selected on the basis of the host cells to be used for expression, which is operatively linked to the nucleic acid sequence to be expressed. Within a recombinant expression vector, "operably linked" is intended to mean that the nucleotide sequence of interest is linked to the regulatory sequence(s) in a manner which allows for expression of the nucleotide sequence *(e.g.,* in an *in vitro* transcription/translation system or in a host cell when the vector is introduced into the host cell). The term "regulatory sequence" is intended to include promoters, enhancers and other expression control elements (*e.g.*, polyadenylation signals). Such regulatory sequences are described, for example, in Goeddel; *Gene Expression Technology: Methods in Enzymology* 185, Academic Press, San Diego, CA (1990). Regulatory sequences include those which direct constitutive expression of a nucleotide sequence in many types of host cells and those which direct expression of the nucleotide sequence only in certain host cells (*e.g.*, tissue-specific regulatory sequences). It will be appreciated by those skilled in the art that the design of the expression vector can depend on such factors as the choice of the host cell to be transformed, the level of expression of protein desired, and the like. The expression vectors of the invention can be introduced into host cells to thereby produce proteins or peptides, including fusion proteins or peptides, encoded by nucleic acids as described herein (*e.g.*, TWIK-8 proteins, mutant forms of TWIK-8 proteins, fusion proteins, and the like).

The recombinant expression vectors of the invention can be designed for expression of TWIK-8 proteins in prokaryotic or eukaryotic cells. For example, TWIK-8 proteins can be expressed in bacterial cells such as *E. coli,* insect cells (using baculovirus expression vectors) yeast cells or mammalian cells. Suitable host cells are discussed further in Goeddel, *Gene Expression Technology: Methods in Enzymology* 185, Academic Press, San Diego, CA (1990). Alternatively, the recombinant expression vector can be transcribed and translated *in vitro,* for example using T7 promoter regulatory sequences and T7 polymerase.

Expression of proteins in prokaryotes is most often carried out in E. *coli* with vectors containing constitutive or inducible promoters directing the expression of either fusion or non-fusion proteins. Fusion vectors add a number of amino acids to a protein encoded therein, usually to the amino terminus of the recombinant protein. Such fusion vectors typically serve three purposes: 1) to increase expression of recombinant protein; 2) to increase the solubility of the recombinant protein; and 3) to aid in the purification of the recombinant protein by acting as a ligand in affinity purification. Often, in fusion expression vectors, a proteolytic cleavage site is introduced at the junction of the fusion moiety and the recombinant protein to enable separation of the recombinant protein from the fusion moiety subsequent to purification of the fusion protein. Such enzymes, and their cognate recognition sequences, include Factor Xa, thrombin and enterokinase. Typical fusion expression vectors include pGEX (Pharmacia Biotech Inc; Smith, D.B. and Johnson, K.S. (1988) *Gene* 67:31-40), pMAL (New England Biolabs, Beverly, MA) and pRIT5 (Pharmacia, Piscataway, NJ) which fuse glutathione S-transferase (GSI), maltose E binding protein, or protein A, respectively, to the target recombinant protein.

Purified fusion proteins can be utilized in TWIK-8 activity assays, (*e.g.*, direct assays or competitive assays described in detail below), or to generate antibodies specific for TWIK-8 proteins, for example. In a preferred embodiment, a TWIK-8 fusion protein expressed in a retroviral expression vector of the present invention can be utilized to infect bone marrow cells which are subsequently transplanted into irradiated recipients. The pathology of the subject recipient is then examined after sufficient time has passed (*e.g.*, six (6) weeks).

Examples of suitable inducible non-fusion *E. coli* expression vectors include pTrc (Amann *et al.,* (1988) *Gene* 69:301-315) and pET 11d (Studier *et al., Gene Expression Technology: Methods in Enzymology* 185, Academic Press, San Diego, California (1990) 60-89). Target gene expression from the pTrc vector relies on host RNA polymerase transcription from a hybrid trp-lac fusion promoter. Target gene expression from the pET 11d vector relies on transcription from a T7 gn10-lac fusion promoter mediated by a coexpressed viral RNA polymerase (T7 gn1). This viral polymerase is supplied by host strains BL21(DE3) or HMS174(DE3) from a resident prophage harboring a T7 gn1 gene under the transcriptional control of the IacUV 5 promoter.

One strategy to maximize recombinant protein expression in E. *coli* is to express the protein in a host bacteria with an impaired capacity to proteolytically cleave the recombinant protein (Gottesman, S., *Gene Expression Technology: Methods in Enzymology* 185, Academic Press, San Diego, California (1990) 119-128). Another strategy is to alter the nucleic acid sequence of the nucleic acid to be inserted into an expression vector so that the individual codons for each amino acid are those preferentially utilized in *E*. *coli* (Wada *et al.,* (1992) *Nucleic Acids Res.* 20:2111-2118). Such alteration of nucleic acid sequences of the invention can be carried out by standard DNA synthesis techniques.

In another embodiment, the TWIK-8 expression vector is a yeast expression vector. Examples of vectors for expression in yeast *S. cerevisiae* include pYepSec1 (Baldari, *et al.,* (1987) *Embo J.* 6:229-234), pMFa (Kurjan and Herskowitz, (1982) *Cell* 30:933-943), pJRY88 (Schultz *et al.,* (1987) *Gene* 54:113-123), pYES2 (Invitrogen Corporation, San Diego, CA), and picZ (InVitrogen Corp, San Diego, CA).

Alternatively, TWIK-8 proteins can be expressed in insect cells using baculovirus expression vectors. Baculovirus vectors available for expression of proteins in cultured insect cells *(e.g.,* Sf 9 cells) include the pAc series (Smith *et al.* (1983) *Mol. Cell Biol.* 3:2156-2165) and the pVL series (Lucklow and Summers (1989) *Virology* 170:31-39).

In yet another embodiment, a nucleic acid of the invention is expressed in mammalian cells using a mammalian expression vector. Examples of mammalian expression vectors include pCDM8 (Seed, B. (1987) *Nature* 329:840) and pMT2PC (Kaufman *et al.* (1987) *EMBO J.* 6:187-195). When used in mammalian cells, the expression vector's control functions are often provided by viral regulatory elements. For example, commonly used promoters are derived from polyoma, Adenovirus 2, cytomegalovirus and Simian Virus 40. For other suitable expression systems for both prokaryotic and eukaryotic cells see chapters 16 and 17 of Sambrook, J., Fritsh, E. F., and Maniatis, T. *Molecular Cloning: A Laboratory Manual. 2nd, ed, Cold Spring Harbor Laboratory,* Cold Spring Harbor Laboratory Press, Cold Spring Harbor, NY, 1989.

In another embodiment, the recombinant mammalian expression vector is capable of directing expression of the nucleic acid preferentially in a particular cell type (*e.g.*, tissue-specific regulatory elements are used to express the nucleic acid). Tissue-specific regulatory elements are known in the art. Non-limiting examples of suitable tissue-specific promoters include the albumin promoter (liver-specific; Pinkert *et al.* (1987) *Genes Dev.* 1:268-277), lymphoid-specific promoters (Calame and Eaton (1988) *Adv. Immunol.* 43:235-275), in particular promoters of T cell receptors (Winoto and Baltimore (1989) *EMBO J.* 8:729-733) and immunoglobulins (Banerji *et al.* (1983) *Cell* 33:729-740; Queen and Baltimore (1983) *Cell* 33:741-748), neuron-specific promoters (*e.g.,* the neurofilament promoter; Byrne and Ruddle (1989) *Proc. Natl. Acad. Sci. USA* 86:5473-5477), pancreas-specific promoters (Edlund *et al.* (1985) *Science* 230:912-916), and mammary gland-specific promoters (*e.g.*, milk whey promoter; U.S. Patent No. 4,873,316 and European Application Publication No. 264,166). Developmentally-regulated promoters are also encompassed, for example the murine hox promoters (Kessel and Gruss (1990) *Science* 249:374-379) and the α-fetoprotein promoter (Campes and Tilghman (1989) *Genes Dev.* 3:537-546).

The invention further provides a recombinant expression vector comprising a DNA molecule of the invention cloned into the expression vector in an antisense orientation. That is, the DNA molecule is operatively linked to a regulatory sequence in a manner which allows for expression (by transcription of the DNA molecule) of an RNA molecule which is antisense to TWIK-8 mRNA. Regulatory sequences operatively linked to a nucleic acid cloned in the antisense orientation can be chosen which direct the continuous expression of the antisense RNA molecule in a variety of cell types, for instance viral promoters and/or enhancers, or regulatory sequences can be chosen which direct constitutive, tissue specific or cell type specific expression of antisense RNA. The antisense expression vector can be in the form of a recombinant plasmid, phagemid or attenuated virus in which antisense nucleic acids are produced under the control of a high efficiency regulatory region, the activity of which can be determined by the cell type into which the vector is introduced. For a discussion of the regulation of gene expression using antisense genes see Weintraub, H. *et al.,* Antisense RNA as a molecular tool for genetic analysis, *Reviews - Trends in Genetics,* Vol. 1(1) 1986.

Another aspect of the invention pertains to host cells into which a TWIK-8 nucleic acid molecule of the invention is introduced, *e.g.*, a TWIK-8 nucleic acid molecule within a recombinant expression vector or a TWIK-8 nucleic acid molecule containing sequences which allow it to homologously recombine into a specific site of the host cell's genome. The terms "host cell" and "recombinant host cell" are used interchangeably herein. It is understood that such terms refer not only to the particular subject cell but to the progeny or potential progeny of such a cell. Because certain modifications may occur in succeeding generations due to either mutation or environmental influences, such progeny may not, in fact, be identical to the parent cell, but are still included within the scope of the term as used herein.

A host cell can be any prokaryotic or eukaryotic cell. For example, a TWIK-8 protein can be expressed in bacterial cells such as *E. coli,* insect cells, yeast or mammalian cells (such as Chinese hamster ovary cells (CHO) or COS cells). Other suitable host cells are known to those skilled in the art.

Vector DNA can be introduced into prokaryotic or eukaryotic cells via conventional transformation or transfection techniques. As used herein, the terms "transformation" and "transfection" are intended to refer to a variety of art-recognized techniques for introducing foreign nucleic acid (*e.g.*, DNA) into a host cell, including calcium phosphate or calcium chloride co-precipitation, DEAE-dextran-mediated transfection, lipofection, or electroporation. Suitable methods for transforming or transfecting host cells can be found in Sambrook, *et al. (Molecular Cloning: A Laboratory Manual. 2nd, ed., Cold Spring Harbor Laboratory,* Cold Spring Harbor Laboratory Press, Cold Spring Harbor, NY, 1989), and other laboratory manuals.

For stable transfection of mammalian cells, it is known that, depending upon the expression vector and transfection technique used, only a small fraction of cells may integrate the foreign DNA into their genome. In order to identify and select these integrants, a gene that encodes a selectable marker (*e.g.*, resistance to antibiotics) is generally introduced into the host cells along with the gene of interest. Preferred selectable markers include those which confer resistance to drugs, such as G418, hygromycin and methotrexate. Nucleic acid encoding a selectable marker can be introduced into a host cell on the same vector as that encoding a TWIK-8 protein or can be introduced on a separate vector. Cells stably transfected with the introduced nucleic acid can be identified by drug selection (*e.g.*, cells that have incorporated the selectable marker gene will survive, while the other cells die).

A host cell of the invention, such as a prokaryotic or eukaryotic host cell in culture, can be used to produce (*i.e.,* express) a TWIK-8 protein. Accordingly, the invention further provides methods for producing a TWIK-8 protein using the host cells of the invention. In one embodiment, the method comprises culturing the host cell of the invention (into which a recombinant expression vector encoding a TWIK-8 protein has been introduced) in a suitable medium such that a TWIK-8 protein is produced. In another embodiment, the method further comprises isolating a TWIK-8 protein from the medium or the host cell.

IV. Pharmaceutical Compositions Further, an antibody (or fragment thereof) may be conjugated to a therapeutic moiety such as a cytotoxin, a therapeutic agent or a radioactive metal ion. A cytotoxin or cytotoxic agent includes any agent that is detrimental to cells. Examples include taxol, cytochalasin B, gramicidin D, ethidium bromide, emetine, mitomycin, etoposide, tenoposide, vincristine, vinblastine, colchicin, doxorubicin, daunorubicin, dihydroxy anthracin dione, mitoxantrone, mithramycin, actinomycin D, 1-dehydrotestosterone, glucocorticoids, procaine, tetracaine, lidocaine, propranolol, and puromycin and analogs or homologs thereof. Therapeutic agents include, but are not limited to, antimetabolites (*e.g.*, methotrexate, 6-mercaptopurine, 6-thioguanine, cytarabine, 5-fluorouracil decarbazine), alkylating agents (*e.g.*, mechlorethamine, thioepa chlorambucil, melphalan, carmustine (BSNU) and lomustine (CCNU), cyclothosphamide, busulfan, dibromomannitol, streptozotocin, mitomycin C, and cis-dichlorodiamine platinum (II) (DDP) cisplatin), anthracyclines (*e.g*., daunorubicin (formerly daunomycin) and doxorubicin), antibiotics (*e.g*., dactinomycin (formerly actinomycin), bleomycin, mithramycin, and anthramycin (AMC)), and anti-mitotic agents (*e.g.,* vincristine and vinblastine).

The conjugates of the invention can be used for modifying a given biological response, the drug moiety is not to be construed as limited to classical chemical therapeutic agents. For example, the drug moiety may be a protein or polypeptide possessing a desired biological activity. Such proteins may include, for example, a toxin such as abrin, ricin A, pseudomonas exotoxin, or diphtheria toxin; a protein such as tumor necrosis factor, alpha-interferon, beta-interferon, nerve growth factor, platelet derived growth factor, tissue plasminogen activator; or, biological response modifiers such as, for example, lymphokines, interleukin-1 ("IL-1"), interleukin-2 ("IL-2"), interleukin-6 ("IL-6"), granulocyte macrophage colony stimulating factor ("GM-CSF"), granulocyte colony stimulating factor ("G-CSF"), or other growth factors.

Techniques for conjugating such therapeutic moiety to antibodies are well known, see, *e.g.,* Arnon *et al.,* "Monoclonal Antibodies For Immunotargeting Of Drugs In Cancer Therapy", in Monoclonal Antibodies And Cancer Therapy, Reisfeld *et al.* (eds.), pp. 243-56 (Alan R. Liss, Inc. 1985); Hellstrom *et al.,* "Antibodies For Drug Delivery", in Controlled Drug Delivery (2nd Ed.), Robinson *et al.* (eds.), pp. 623-53 (Marcel Dekker, Inc. 1987); Thorpe, "Antibody Carriers Of Cytotoxic Agents In Cancer Therapy: A Review", in Monoclonal Antibodies '84: Biological And Clinical Applications, Pinchera *et al.* (eds.), pp. 475-506 (1985); "Analysis, Results, And Future Prospective Of The Therapeutic Use Of Radiolabeled Antibody In Cancer Therapy", in Monoclonal Antibodies For Cancer Detection And Therapy, Baldwin *et al.* (eds.), pp. 303-16 (Academic Press 1985), and Thorpe *et al.,* "The Preparation And Cytotoxic Properties Of Antibody-Toxin Conjugates", Immunol. Rev., 62:119-58 (1982). Alternatively, an antibody can be conjugated to a second antibody to form an antibody heteroconjugate as described by Segal in U.S. Patent No. 4,676,980.

### V. Uses and Methods of the Invention

The nucleic acid molecules, proteins, protein homologues, and antibodies described herein can be used in one or more of the following methods: a) screening assays; b) predictive medicine (*e.g.*, diagnostic assays,). As described herein, a TWIK-8 protein of the invention has one or more of the following activities: (1) interacting with a non-TWIK protein molecule; (2) activating a TWIK-dependent signal transduction pathway; (3) modulating the release of neurotransmitters; (4) modulating membrane excitability; (5) influencing the resting potential of membranes, wave forms and frequencies of action potentials, and thresholds of excitation, (6) modulating processes which underlie learning and memory, such as integration of sub-threshold synaptic responses and the conductance of back-propagating action potentials, and (7) mediating nociception.

The isolated nucleic acid molecules of the invention can be used, for example, to express TWIK-8 protein (*e.g.*, via a recombinant expression vector in a host cell in gene therapy applications), to detect TWIK-8 mRNA (*e.g.*, in a biological sample) or a genetic alteration in a TWIK-8 gene, and to modulate TWIK-8 activity, as described further below. In addition, the TWIK-8 proteins can be used to screen for naturally occurring TWIK-8 substrates, to screen for drugs or compounds which modulate TWIK-8 activity.

### A. Screening Assays:

The invention provides a method (also referred to herein as a "screening assay") for identifying modulators, *i.e.,* candidate or test compounds or agents (*e.g.*, peptides, peptidomimetics, small molecules or other drugs) which bind to TWIK-8 proteins, have a stimulatory or inhibitory effect on, for example, TWIK-8 expression or TWIK-8 activity, or have a stimulatory or inhibitory effect on, for example, the expression or activity of TWIK-8 substrate.

In one embodiment, the invention provides assays for screening candidate or test compounds which are substrates of a TWIK-8 protein or polypeptide or biologically active portion thereof. In another embodiment, the invention provides assays for screening candidate or test compounds which bind to or modulate the activity of a TWIK-8 protein or polypeptide or biologically active portion thereof. The test compounds of the present invention can be obtained using any of the numerous approaches in combinatorial library methods known in the art, including: biological libraries; spatially addressable parallel solid phase or solution phase libraries; synthetic library methods requiring deconvolution; the 'one-bead one-compound' library method; and synthetic library methods using affinity chromatography selection. The biological library approach is limited to peptide libraries, while the other four approaches are applicable to peptide, non-peptide oligomer or small molecule libraries of compounds (Lam, K.S. (1997) *Anticancer Drug Des.* 12:145).

Examples of methods for the synthesis of molecular libraries can be found in the art, for example in: DeWitt *et al.* (1993) *Proc. Natl. Acad. Sci. U.S.A.* 90:6909; Erb *et al.* (1994) *Proc. Natl. Acad. Sci. USA* 91:11422; Zuckermann *et al.* (1994). *J. Med Chem.* 37:2678; Cho *et al.* (1993) *Science* 261:1303; Carrell *et al.* (1994) *Angew. Chem. Int. Ed. Engl.* 33:2059; Carell *et al. (1994) Angew. Chem. Int. Ed Engl.* 33:2061; and in Gallop *et al.* (1994) *J. Med Chem.* 37:1233.

Libraries of compounds may be presented in solution (*e.g.*, Houghten (1992) *Biotechniques* 13:412-421), or on beads (Lam (1991) *Nature* 354:82-84), chips (Fodor (1993) *Nature* 364:555-556), bacteria (Ladner USP 5,223,409), spores (Ladner USP '409), plasmids (Cull *et al.* (1992) *Proc Natl Acad Sci USA* 89:1865-1869) or on phage (Scott and Smith (1990) *Science* 249:386-390); (Devlin (1990) *Science* 249:404-406); (Cwirla *et al. (1990) Proc. Natl. Acad Sci.* 87:6378-6382); (Felici (1991) *J. Mol. Biol.* 222:301-310); (Ladner *supra.*).

In one embodiment, an assay is a cell-based assay in which a cell which expresses a TWIK-8 protein or biologically active portion thereof is contacted with a test compound and the ability of the test compound to modulate TWIK-8 activity is determined. Determining the ability of the test compound to modulate TWIK-8 activity can be accomplished by monitoring, for example, the release of a neurotransmitter from a cell which expresses TWIK-8. The cell, for example, can be of mammalian origin, *e.g.*, a neuronal cell, cardiac cell or a skin cell.

The ability of the test compound to modulate TWIK-8 binding to a substrate or to bind to TWIK-8 can also be determined. Determining the ability of the test compound to modulate TWIK-8 binding to a substrate can be accomplished, for example, by coupling the TWIK-8 substrate with a radioisotope or enzymatic label such that binding of the TWIK-8 substrate to TWIK-8 can be determined by detecting the labeled TWIK-8 substrate in a complex. Alternatively, TWIK-8 could be coupled with a radioisotope or enzymatic label to monitor the ability of a test compound to modulate TWIK-8 binding to a TWIK-8 substrate in a complex. Determining the ability of the test compound to bind TWIK-8 can be accomplished, for example, by coupling the compound with a radioisotope or enzymatic label such that binding of the compound to TWIK-8 can be determined by detecting the labeled TWIK-8 compound in a complex. For example, compounds (*e.g.,* TWIK-8 substrates) can be labeled with ¹²⁵I, ³⁵S, ¹⁴C, or ³H, either directly or indirectly, and the radioisotope detected by direct counting of radioemission or by scintillation counting. Alternatively, compounds can be enzymatically labeled with, for example, horseradish peroxidase, alkaline phosphatase, or luciferase, and the enzymatic label detected by determination of conversion of an appropriate substrate to product.

It is also within the scope of this invention to determine the ability of a compound (*e.g.*, a TWIK-8 substrate) to interact with TWIK-8 without the labeling of any of the interactants. For example, a microphysiometer can be used to detect the interaction of a compound with TWIK-8 without the labeling of either the compound or the TWIK-8. McConnell, H. M. *et al.* (1992) *Science* 257:1906-1912. As used herein, a "microphysiometer" (*e.g.*, Cytosensor) is an analytical instrument that measures the rate at which a cell acidifies its environment using a light-addressable potentiometric sensor (LAPS). Changes in this acidification rate can be used as an indicator of the interaction between a compound and TWIK-8.

In another embodiment, an assay is a cell-based assay comprising contacting a cell expressing a TWIK-8 target molecule (*e.g.,* a TWIK-8 substrate) with a test compound and determining the ability of the test compound to modulate (*e.g.*, stimulate or inhibit) the activity of the TWIK-8 target molecule. Determining the ability of the test compound to modulate the activity of a TWIK-8 target molecule can be accomplished, for example, by determining the ability of the TWIK-8 protein to bind to or interact with the TWIK-8 target molecule.

Determining the ability of the TWIK-8 protein, or a biologically active fragment thereof, to bind to or interact with a TWIK-8 target molecule can be accomplished by one of the methods described above for determining direct binding. In a preferred embodiment, determining the ability of the TWIK-8 protein to bind to or interact with a TWIK-8 target molecule can be accomplished by determining the activity of the target molecule. For example, the activity of the target molecule can be determined by detecting induction of a cellular response (*i.e.,* changes in intracellular K⁺ levels), detecting catalytic/enzymatic activity of the target on an appropriate substrate, detecting the induction of a reporter gene (comprising a target-responsive regulatory element operatively linked to a nucleic acid encoding a detectable marker, *e.g*., luciferase), or detecting a target-regulated cellular response.

An assay is a cell-free assay in which a TWIK-8 protein or biologically active portion thereof is contacted with a test compound and the ability of the test compound to bind to the TWIK-8 protein or biologically active portion thereof is determined. Preferred biologically active portions of the TWIK-8 proteins to be used in assays include fragments which participate in interactions with non-TWIK-8 molecules, *e.g.*, fragments with high surface probability scores. Binding of the test compound to the TWIK-8 protein can be determined either directly or indirectly as described above. In a preferred embodiment, the assay includes contacting the TWIK-8 protein or biologically active portion thereof with a known compound which binds TWIK-8 to form an assay mixture, contacting the assay mixture with a test compound, and determining the ability of the test compound to interact with a TWIK-8 protein, wherein determining the ability of the test compound to interact with a TWIK-8 protein comprises determining the ability of the test compound to preferentially bind to TWIK-8 or biologically active portion thereof as compared to the known compound.

In another embodiment, the assay is a cell-free assay in which a TWIK-8 protein or biologically active portion thereof is contacted with a test compound and the ability of the test compound to modulate (*e.g.,* stimulate or inhibit) the activity of the TWIK-8 protein or biologically active portion thereof is determined. Determining the ability of the test compound to modulate the activity of a TWIK-8 protein can be accomplished, for example, by determining the ability of the TWIK-8 protein to bind to a TWIK-8 target molecule by one of the methods described above for determining direct binding. Determining the ability of the TWIK-8 protein to bind to a TWIK-8 target molecule can also be accomplished using a technology such as real-time Biomolecular Interaction Analysis (BIA). Sjolander, S. and Urbaniczky, C. (1991) *Anal. Chem.* 63:2338-2345 and Szabo *et al.* (1995) *Curr. Opin. Struct. Biol.* 5:699-705. As used herein, "BIA" is a technology for studying biospecific interactions in real time, without labeling any of the interactants (*e.g.*, BlAcore). Changes in the optical phenomenon of surface plasmon resonance (SPR) can be used as an indication of real-time reactions between biological molecules.

In an alternative embodiment, determining the ability of the test compound to modulate the activity of a TWIK-8 protein can be accomplished by determining the ability of the TWIK-8 protein to further modulate the activity of a downstream effector of a TWIK-8 target molecule. For example, the activity of the effector molecule on an appropriate target can be determined or the binding of the effector to an appropriate target can be determined as previously described.

In yet another embodiment, the cell-free assay involves contacting a TWIK-8 protein or biologically active portion thereof with a known compound which binds the TWIK-8 protein to form an assay mixture, contacting the assay mixture with a test compound, and determining the ability of the test compound to interact with the TWIK-8 protein, wherein determining the ability of the test compound to interact with the TWIK-8 protein comprises determining the ability of the TWIK-8 protein to preferentially bind to or modulate the activity of a TWIK-8 target molecule.

In more than one embodiment of the above assay methods, it may be desirable to immobilize either TWIK-8 or its target molecule to facilitate separation of complexed from uncomplexed forms of one or both of the proteins, as well as to accommodate automation of the assay. Binding of a test compound to a TWIK-8 protein, or interaction of a TWIK-8 protein with a target molecule in the presence and absence of a candidate compound, can be accomplished in any vessel suitable for containing the reactants. Examples of such vessels include microtitre plates, test tubes, and micro-centrifuge tubes. In one embodiment, a fusion protein can be provided which adds a domain that allows one or both of the proteins to be bound to a matrix. For example, glutathione-S-transferase/TWIK-8 fusion proteins or glutathione-S-transferase/target fusion proteins can be adsorbed onto glutathione sepharose beads (Sigma Chemical, St. Louis, MO) or glutathione derivatized microtitre plates, which are then combined with the test compound or the test compound and either the non-adsorbed target protein or TWIK-8 protein, and the mixture incubated under conditions conducive to complex formation (*e.g*., at physiological conditions for salt and pI-n. Following incubation, the beads or microtitre plate wells are washed to remove any unbound components, the matrix immobilized in the case of beads, complex determined either directly or indirectly, for example, as described above. Alternatively, the complexes can be dissociated from the matrix, and the level of TWIK-8 binding or activity determined using standard techniques.

Other techniques for immobilizing proteins on matrices can also be used in the screening assays of the invention. For example, either a TWIK-8 protein or a TWIK-8 target molecule can be immobilized utilizing conjugation of biotin and streptavidin. Biotinylated TWIK-8 protein or target molecules can be prepared from biotin-NHS (N-hydroxy-succinimide) using techniques known in the art (*e.g.,* biotinylation kit, Pierce Chemicals, Rockford, IL), and immobilized in the wells of streptavidin-coated 96 well plates (Pierce Chemical). Alternatively, antibodies reactive with TWIK-8 protein or target molecules but which do not interfere with binding of the TWIK-8 protein to its target molecule can be derivatized to the wells of the plate, and unbound target or TWIK-8 protein trapped in the wells by antibody conjugation. Methods for detecting such complexes, in addition to those described above for the GST-immobilized complexes, include immunodetection of complexes using antibodies reactive with the TWIK-8 protein or target molecule, as well as enzyme-linked assays which rely on detecting an enzymatic activity associated with the TWIK-8 protein or target molecule.

In another embodiment, modulators of TWIK-8 expression are identified in a method wherein a cell is contacted with a candidate compound and the expression of TWIK-8 mRNA or protein in the cell is determined. The level of expression of TWIK-8 mRNA or protein in the presence of the candidate compound is compared to the level of expression of TWIK-8 mRNA or protein in the absence of the candidate compound. The candidate compound can then be identified as a modulator of TWIK-8 expression based on this comparison. For example, when expression of TWIK-8 mRNA or protein is greater (statistically significantly greater) in the presence of the candidate compound than in its absence, the candidate compound is identified as a stimulator of TWIK-8 mRNA or protein expression. Alternatively, when expression of TWIK-8 mRNA or protein is less (statistically significantly less) in the presence of the candidate compound than in its absence, the candidate compound is identified as an inhibitor of TWIK-8 mRNA or protein expression. The level of TWIK-8 mRNA or protein expression in the cells can be determined by methods described herein for detecting TWIK-8 mRNA or protein.

In yet another aspect, the TWIK-8 proteins can be used as "bait proteins" in a two-hybrid assay or three-hybrid assay (see, *e.g.*, U.S. Patent No. 5,283,317; Zervos *et al.* (1993) *Cell* 72:223-232; Madura *et al.* (1993) *J. Biol. Chem.* 268:12046-12054; Bartel *et al.* (1993) *Biotechniques* 14:920-924; Iwabuchi *et al.* (1993) *Oncogene* 8:1693-1696; and Brent WO94/10300), to identify other proteins, which bind to or interact with TWIK-8 ("TWIK-8-binding proteins" or "TWIK-8-bp") and are involved in TWIK-8 activity. Such TWIK-8-binding proteins are also likely to be involved in the propagation of signals by the TWIK-8 proteins or TWIK-8 targets as, for example, downstream elements of a TWIK-8-mediated signaling pathway. Alternatively, such TWIK-8-binding proteins are likely to be TWIK-8 inhibitors.

The two-hybrid system is based on the modular nature of most transcription factors, which consist of separable DNA-binding and activation domains. Briefly, the assay utilizes two different DNA constructs. In one construct, the gene that codes for a TWIK-8 protein is fused to a gene encoding the DNA binding domain of a known transcription factor (*e.g.*, GAL-4). In the other construct, a DNA sequence, from a library of DNA sequences, that encodes an unidentified protein ("prey" or "sample") is fused to a gene that codes for the activation domain of the known transcription factor. If the "bait" and the "prey" proteins are able to interact, *in vivo,* forming a TWIK-8-dependent complex, the DNA-binding and activation domains of the transcription factor are brought into close proximity. This proximity allows transcription of a reporter gene (*e.g*., LacZ) which is operably linked to a transcriptional regulatory site responsive to the transcription factor. Expression of the reporter gene can be detected and cell colonies containing the functional transcription factor can be isolated and used to obtain the cloned gene which encodes the protein which interacts with the TWIK-8 protein.

In another aspect, the disclosure pertains to a combination of two or more of the assays described herein. For example, a modulating agent can be identified using a cell-based or a cell free assay, and the ability of the agent to modulate the activity of a TWIK-8 protein can be confirmed *in vivo, e.g.,* in an animal such as an animal model for pain.

### B. Detection Assays

Portions or fragments of the cDNA sequences identified herein (and the corresponding complete gene sequences) can be used in numerous ways as polynucleotide reagents. For example, these sequences can be used to: (i) map their respective genes on a chromosome; and, thus, locate gene regions associated with genetic disease; (ii) identify an individual from a minute biological sample (tissue typing); and (iii) aid in forensic identification of a biological sample. These applications are described in the subsections below.

### C. Monitoring of Effects During Clinical Trials

Monitoring the influence of agents (*e.g.*, drugs) on the expression or activity of a TWIK-8 protein (*e.g.*, the modulation of pain signaling mechanisms, neurotransmitter release and/or membrane excitability) can be applied not only in basic drug screening, but also in clinical trials. For example, the effectiveness of an agent determined by a screening assay as described herein to increase TWIK-8 gene expression, protein levels, or upregulate TWIK-8 activity, can be monitored in clinical trials of subjects exhibiting decreased TWIK-8 gene expression, protein levels, or downregulated TWIK-8 activity. Alternatively, the effectiveness of an agent determined by a screening assay to decrease TWIK-8 gene expression, protein levels, or downregulate TWIK-8 activity, can be monitored in clinical trials of subjects exhibiting increased TWIK-8 gene expression, protein levels, or upregulated TWIK-8 activity. In such clinical trials, the expression or activity of a TWIK-8 gene, and preferably, other genes that have been implicated in, for example, a TWIK-8-associated disorder can be used as a "read out" or markers of the phenotype of a particular cell.

For example, and not by way of limitation, genes, including TWIK-8, that are modulated in cells by treatment with an agent (*e.g.*, compound, drug or small molecule) which modulates TWIK-8 activity (*e.g.*, identified in a screening assay as described herein) can be identified. Thus, to study the effect of agents on TWIK-8-associated disorders (*e.g.*, disorders characterized by deregulated pain signaling mechanisms, neurotransmitter release and/or membrane excitability), for example, in a clinical trial, cells can be isolated and RNA prepared and analyzed for the levels of expression of TWIK-8 and other genes implicated in the TWIK-8-associated disorder, respectively. The levels of gene expression (*e.g.*, a gene expression pattern) can be quantified by northern blot analysis or RT-PCR, as described herein, or alternatively by measuring the amount of protein produced, by one of the methods as described herein, or by measuring the levels of activity of TWIK-8 or other genes. In this way, the gene expression pattern can serve as a marker, indicative of the physiological response of the cells to the agent. Accordingly, this response state may be determined before, and at various points during treatment of the individual with the agent.

In a preferred embodiment, the present invention provides a method for monitoring the effectiveness of treatment of a subject with an agent (*e.g.*, an agonist, antagonist, peptidomimetic, protein, peptide, nucleic acid, small molecule, or other drug candidate identified by the screening assays described herein) including the steps of (i) obtaining a pre-administration sample from a subject prior to administration of the agent; (ii) detecting the level of expression of a TWIK-8 protein, mRNA, or genomic DNA in the preadministration sample; (iii) obtaining one or more post-administration samples from the subject; (iv) detecting the level of expression or activity of the TWIK-8 protein, mRNA, or genomic DNA in the post-administration samples; (v) comparing the level of expression or activity of the TWIK-8 protein, mRNA, or genomic DNA in the pre-administration sample with the TWIK-8 protein, mRNA, or genomic DNA in the post administration sample or samples; and (vi) altering the administration of the agent to the subject accordingly. For example, increased administration of the agent may be desirable to increase the expression or activity of TWIK-8 to higher levels than detected, *i.e.,* to increase the effectiveness of the agent. Alternatively, decreased administration of the agent may be desirable to decrease expression or activity of TWIK-8 to lower levels than detected, i. e. to decrease the effectiveness of the agent. According to such an embodiment, TWIK-8 expression or activity may be used as an indicator of the effectiveness of an agent, even in the absence of an observable phenotypic response.

### D Therapeutic Methods

Another aspect of the invention pertains to methods of modulating TWIK-8 expression or activity for therapeutic purposes. Accordingly, the modulatory method of the invention involves contacting a cell with a TWIK-8 or agent that modulates one or more of the activities of TWIK-8 protein activity associated with the cell. Said agent that modulates TWIK-8 protein activity is an antibody, or an immunologically active portion thereof, which selectively binds to TWIK-8. These modulatory methods can be performed *in vitro* (*e.g.,* by culturing the cell with the antibody) or, alternatively, *in vivo* (*e.g.,* by administering the antibody to a subject). As such, the present invention provides methods of treating an individual afflicted with a disease or disorder characterized by aberrant or unwanted expression or activity of a TWIK-8 protein or nucleic acid molecule. In one embodiment, the method involves administering an antibody.

Stimulation of TWIK-8 activity is desirable in situations in which TWIK-8 is abnormally downregulated and/or in which increased TWIK-8 activity is likely to have a beneficial effect. Likewise, inhibition of TWIK-8 activity is desirable in situations in which TWIK-8 is abnormally upregulated and/or in which decreased TWIK-8 activity is likely to have a beneficial effect.

### EXAMPLES

### EXAMPLE 1: IDENTIFICATION AND CHARACTERIZATION OF HUMAN TWIK-8 cDNA

In this example, the identification and characterization of the gene encoding human TWIK-8 (clone Fbh12303) is described.

### Isolation of the TWIK-8 cDNA

The invention is based, at least in part, on the discovery of a human gene encoding a novel protein, referred to herein as TWIK-8. The entire sequence of the human clone Fbh12303 was determined and found to contain an open reading frame termed human "TWIK-8."

The nucleotide sequence encoding the human TWIK-8 protein is shown in Figure 1 and is set forth as SEQ ID NO:1. The protein encoded by this nucleic acid comprises about 419 amino acids and has the amino acid sequence shown in Figure 1 and set forth as SEQ ID NO:2. The coding region (open reading frame) of SEQ ID NO:1 is set forth as SEQ ID NO:3.

### Analysis of the Human TWIK-8 Molecule

The amino acid sequence of human TWIK-8 was analyzed using the program PSORT (http://www. psort.nibb.ac.jp) to predict the localization of the proteins within the cell. This program assesses the presence of different targeting and localization amino acid sequences within the query sequence. The results of the analysis show that human TWIK-8 (SEQ ID NO:2) may be localized to the endoplasmic reticulum or to the mitochondrion.

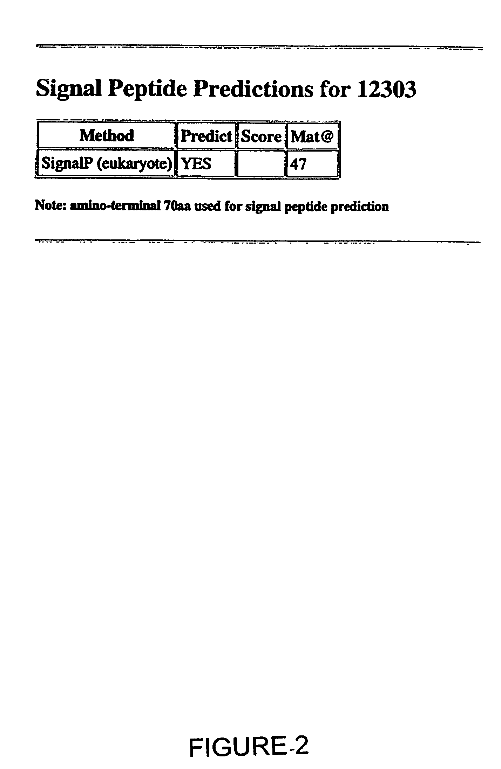

An analysis of the amino acid sequence of human TWIK-8 using the Signal P program (Henrik, *et al.* (1997) *Protein Engineering* 10:1-6), identified the presence of a signal peptide from amino acids 1-46 (Figure 2).

A search was performed against the Memsat database (Figure 3) resulting in the identification of six transmembrane domains in the amino acid sequence of the native human TWIK-8 (SEQ ID NO: 2) at about residues 32-50, 116-137, 144-165, 195-219, 226-242, and 260-283. This search further identified five transmembrane domains in the amino acid sequence of the predicted mature form of this protein, at about residues 70-91, 98-119, 149-173, 180-196, and 214-237. The results of this search are set forth in Figure 3.

A search was performed against the HMM database (Figure 4), resulting in the identification of a "seven-transmembrane receptor domain" from about residues 25-244, and a "cyclic nucleotide-gated channel domain" from about residues 27-204 in the amino acid sequence of human TWIK-8 (SEQ ID NO:2).

A search was also performed against the ProDom database (Figure 5), resulting in the identification of "TRAAK potassium channel domains" from about residues 50-104 (score= 175), 175-199 (score = 115), and 288-382 (score =135); a "potassium channel protein domain" from about residues 99-153 (score =101); a "voltage-gated potassium channel domain" from about residues 102-168 (score = 115); an "outward-rectifier TOK1 potassium channel domain" from about residues 215-270 (score = 70); and a "potassium channel subunit domain" from about residues 216-287 (score = 156) in the amino acid sequence of human TWIK-8 (SEQ ID NO:2).

A BLASTX 2.0 search against the NRP/protot database, using a score of 100, a wordlength of 3, and a Blosum 62 matrix (Altschul *et al.* (1990) *J. Mol. Biol.* 215:403), of the translated nucleotide sequence of human TWIK-8 revealed that human TWIK-8 has limited sequence homology to *Mus musculus* TRAAK K+ channel subunit mRNA (Accession Number AF056492), to *Homo sapiens* TREK-1 potassium channel (KCNK2) mRNA (Accession Number AF129399), to *Mus musculus* TREK-1 K+ channel subunit mRNA (Accession Number U73488), and to *Homo sapiens* two-pore potassium channel TPKC1 mRNA (Accession number AF004711).

### Tissue Distribution of TWIK-8 mRNA by PCR and In Situ Hybridization

The tissue distribution of TWIK-8 mRNA is determined by Polymerase Chain Reaction (PCR) on cDNA libraries of normal human tissues using oligonucleotide primers based on the human TWIK-8 sequence.

The tissue distribution of TWIK-8 mRNA is also determined using *in situ* hybridization. For *in situ* analysis, various tissues, *e.g*. tissues obtained from brain, are first frozen on dry ice. Ten-micrometer-thick sections of the tissues are postfixed with 4% formaldehyde in DEPC treated 1X phosphate- buffered saline at room temperature for 10 minutes before being rinsed twice in DEPC 1X phosphate-buffered saline and once in 0.1 M triethanolamine-HCl (pH 8.0). Following incubation in 0.25% acetic anhydride-0.1 M triethanolamine-HCl for 10 minutes, sections are rinsed in DEPC 2X SSC (1X SSC is 0.15M NaCl plus 0.015M sodium citrate). Tissue is then dehydrated through a series of ethanol washes, incubated in 100% chloroform for 5 minutes, and then rinsed in 100% ethanol for 1 minute and 95% ethanol for 1 minute and allowed to air dry.

Hybridizations are performed with ³⁵S-radiolabeled (5 X 10⁷ cpm/ml) cRNA probes. Probes are incubated in the presence of a solution containing 600 mM NaCl, 10 mM Tris (pH 7.5), 1 mM EDTA, 0.0 1 % sheared salmon sperm DNA, 0.0 1 % yeast tRNA, 0.05% yeast total RNA type X1, 1X Denhardt's solution, 50% formamide, 10% dextran sulfate, 100 mM dithiothreitol, 0.1% sodium dodecyl sulfate (SDS), and 0.1% sodium thiosulfate for 18 hours at 55°C.

After hybridization, slides are washed with 2X SSC. Sections are then sequentially incubated at 37°C in TNE (a solution containing 10 mM Tris-HCl (pH 7.6), 500 mM NaCl, and 1 mM EDTA), for 10 minutes, in TNE with 10µg of RNase A per ml for 30 minutes, and finally in TNE for 10 minutes. Slides are then rinsed with 2X SSC at room temperature, washed with 2X SSC at 50°C for 1 hour, washed with 0.2X SSC at 55°C for 1 hour, and 0.2X SSC at 60°C for 1 hour. Sections are then dehydrated rapidly through serial ethanol-0.3 M sodium acetate concentrations before being air dried and exposed to Kodak Biomax MR scientific imaging film for 24 hours and subsequently dipped in NB-2 photoemulsion and exposed at 4°C for 7 days before being developed and counter stained.

### Tissue Distribution of human TWIK-8 mRNA using Taqman^{™} analysis

This example describes the tissue distribution of human TWIK-8 mRNA in a variety of cells and tissues, as determined using the TaqMan^{™} procedure. The Taqman^{™} procedure is a quantitative, reverse transcription PCR-based approach for detecting mRNA. The RT-PCR reaction exploits the 5' nuclease activity of AmpliTaq Gold^{™} DNA Polymerase to cleave a TaqMan^{™} probe during PCR. Briefly, cDNA was generated from the samples of interest, *e.g.,* various human tissue samples, and used as the starting material for PCR amplification. In addition to the 5' and 3' gene-specific primers, a gene-specific oligonucleotide probe (complementary to the region being amplified) was included in the reaction (*i.e.,* the Taqman^{™} probe). The TaqMan^{™} probe includes the oligonucleotide with a fluorescent reporter dye covalently linked to the 5' end of the probe (such as FAM (6-carboxyfluorescein), TET (6-carboxy-4,7,2',7'-tetrachlorofluorescein), JOE (6-carboxy-4,5-dichloro-2,7-dimethoxyfluorescein), or VIC) and a quencher dye (TAMRA (6-carboxy-N,N,N',N'-tetramethykhodamine) at the 3' end of the probe.

During the PCR reaction, cleavage of the probe separates the reporter dye and the quencher dye, resulting in increased fluorescence of the reporter. Accumulation of PCR products is detected directly by monitoring the increase in fluorescence of the reporter dye. When the probe is intact, the proximity of the reporter dye to the quencher dye results in suppression of the reporter fluorescence. During PCR, if the target of interest is present, the probe specifically anneals between the forward and reverse primer sites. The 5'-3' nucleolytic activity of the AmpliTaq^{™} Gold DNA Polymerase cleaves the probe between the reporter and the quencher only if the probe hybridizes to the target. The probe fragments are then displaced from the target, and polymerization of the strand continues. The 3' end of the probe is blocked to prevent extension of the probe during PCR. This process occurs in every cycle and does not interfere with the exponential accumulation of product. RNA was prepared using the trizol method and treated with DNase to remove contaminating genomic DNA. cDNA was synthesized using standard techniques. Mock cDNA synthesis in the absence of reverse transcriptase resulted in samples with no detectable PCR amplification of the control gene confirms efficient removal of genomic DNA contamination.

Highest expression of TWIK-8 mRNA was detected in cortex, followed by dorsal root ganglia, and hypothalamus. Weak expression was also detected in erythroid tissue, followed by HUVEC, spinal cord, hermangioma, kidney, normal ovary and ovary tumor, megakaryocytes, normal prostate and prostate tumor. Weak expression was also detected in breast tumor although no expression was detected in normal breast tissue.

### EXAMPLE 2: EXPRESSION OF RECOMBINANT TWIK-8 PROTEIN IN BACTERIAL CELLS

In this example, TWIK-8 is expressed as a recombinant glutathione-S-transferase (GST) fusion polypeptide in E. *coli* and the fusion polypeptide is isolated and characterized. Specifically, TWIK-8 is fused to GST and this fusion polypeptide is expressed in *E. coli, e.g.,* strain PEB199. Expression of the GST-TWIK-8 fusion protein in PEB 199 is induced with IPTG. The recombinant fusion polypeptide is purified from crude bacterial lysates of the induced PEB 199 strain by affinity chromatography on glutathione beads. Using polyacrylamide gel electrophoretic analysis of the polypeptide purified from the bacterial lysates, the molecular weight of the resultant fusion polypeptide is determined.

### EXAMPLE 3: EXPRESSION OF RECOMBINANT TWIK-8 PROTEIN IN COS CELLS

To express the TWIK-8 gene in COS cells, the pcDNA/Amp vector by Invitrogen Corporation (San Diego, CA) is used. This vector contains an SV40 origin of replication, an ampicillin resistance gene, an *E. coli* replication origin, a CMV promoter followed by a polylinker region, and an SV40 intron and polyadenylation site. A DNA fragment encoding the entire TWIK-8 protein and an HA tag (Wilson *et al.* (1984) *Cell* 37:767) or a FLAG tag fused in-frame to its 3' end of the fragment is cloned into the polylinker region of the vector, thereby placing the expression of the recombinant protein under the control of the CMV promoter.

To construct the plasmid, the TWIK-8 DNA sequence is amplified by PCR using two primers. The 5' primer contains the restriction site of interest followed by approximately twenty nucleotides of the TWIK-8 coding sequence starting from the initiation codon; the 3' end sequence contains complementary sequences to the other restriction site of interest, a translation stop codon, the HA tag or FLAG tag and the last 20 nucleotides of the TWIK-8 coding sequence. The PCR amplified fragment and the pCDNA/Amp vector are digested with the appropriate restriction enzymes and the vector is dephosphorylated using the CIAP enzyme (New England Biolabs, Beverly, MA). Preferably the two restriction sites chosen are different so that the TWIK-8 gene is inserted in the correct orientation. The ligation mixture is transformed into *E. coli* cells (strains HB101, DH5α, SURE, available from Stratagene Cloning Systems, La Jolla, CA, can be used), the transformed culture is plated on ampicillin media plates, and resistant colonies are selected. Plasmid DNA is isolated from transformants and examined by restriction analysis for the presence of the correct fragment.

COS cells are subsequently transfected with the TWIK-8-pcDNA/Amp plasmid DNA using the calcium phosphate or calcium chloride co-precipitation methods, DEAE-dextran-mediated transfection, lipofection, or electroporation. Other suitable methods for transfecting host cells can be found in Sambrook, J., Fritsh, E. F., and Maniatis, T. *Molecular Cloning: A Laboratory Manual. 2nd, ed, Cold Spring Harbor Laboratory,* Cold Spring Harbor Laboratory Press, Cold Spring Harbor, NY, 1989. The expression of the TWIK-8 polypeptide is detected by radiolabelling (³⁵S-methionine or ³⁵S-cysteine available from NEN, Boston, MA, can be used) and immunoprecipitation (Harlow, E. and Lane, D. *Antibodies: A Laboratory Manual,* Cold Spring Harbor Laboratory Press, Cold Spring Harbor, NY, 1988) using an HA specific monoclonal antibody. Briefly, the cells are labelled for 8 hours with ³⁵S-methionine (or ³⁵S-cysteine). The culture media are then collected and the cells are lysed using detergents (RIPA buffer, 150 mM NaCl, 1% NP-40, 0.1% SDS, 0.5% DOC, 50 mM Tris, pH 7.5). Both the cell lysate and the culture media are precipitated with an HA specific monoclonal antibody. Precipitated polypeptides are then analyzed by SDS-PAGE.

Alternatively, DNA containing the TWIK-8 coding sequence is cloned directly into the polylinker of the pCDNA/Amp vector using the appropriate restriction sites. The resulting plasmid is transfected into COS cells in the manner described above, and the expression of the TWIK-8 polypeptide is detected by radiolabelling and immunoprecipitation using a TWIK-8 specific monoclonal antibody.

## Claims

1. An isolated nucleic acid molecule selected from the group consisting of:
(a) a nucleic acid molecule comprising the nucleotide sequence set forth in SEQ ID NO:1;
(b) a nucleic acid molecule comprising the nucleotide sequence set forth in SEQ ID NO:3;
(c) an isolated nucleic acid molecule which encodes a polypeptide comprising the amino acid sequence set forth in SEQ ID NO: 2;
(d) an isolated nucleic acid molecule which encodes a naturally occurring allelic variant of a polypeptide comprising the amino acid sequence set forth in SEQ ID NO: 2;
(e) a nucleic acid molecule comprising a nucleotide sequence which is at least 95% identical to the nucleotide sequence of SEQ ID NO:1 or 3, or a complement thereof; and
(f) a nucleic acid molecule which encodes a polypeptide comprising an amino acid sequence at least 95% identical to the amino acid sequence of SEQ ID NO:2.

2. An isolated nucleic acid molecule which comprises a nucleotide sequence that is complementary to the nucleotide sequence of the nucleic acid molecule of claim 1.

3. An isolated nucleic acid molecule comprising the nucleic acid molecule of claim 1 and a nucleotide sequence encoding a heterologous polypeptide.

4. A vector comprising the nucleic acid molecule of claim 1, optionally wherein the vector is an expression vector.

5. A host cell transfected with the expression vector of claim 4.

6. A method of producing a polypeptide comprising culturing the host cell of claim 5 in an appropriate culture medium to, thereby, produce the polypeptide.

7. An isolated polypeptide selected from the group consisting of:
a) a naturally occurring allelic variant of a polypeptide comprising the amino acid sequence of SEQ ID NO:2, wherein the polypeptide is encoded by a nucleic acid molecule which hybridizes to a nucleic acid molecule consisting of SEQ ID NO:1 or 3 under stringent conditions;
b) a polypeptide which is encoded by a nucleic acid molecule comprising a nucleotide sequence which is at least 95% identical to a nucleic acid comprising the nucleotide sequence of SEQ ID NO:1 or 3, and wherein the polypeptide comprises an amino acid sequence which is at least 95% identical to the amino acid of SEQ ID NO: 2;
(c) a polypeptide comprising an amino acid sequence which is at least 95% identical to the amino acid sequence of SEQ ID NO: 2; and
d) a polypeptide comprising the amino acid sequence of SEQ ID NO:2.

8. The polypeptide of claim 7, further comprising heterologous amino acid sequences.

9. An antibody, or an immunologically active portion thereof which selectively binds to a polypeptide of claim 7.

10. The antibody or immunologically active portion thereof of claim 9 which is selected from the group consisting of:
(a) polyclonal antibodies;
(b) monoclonal antibodies;
(c) F(ab) fragments; and
(d) F(ab')z fragments.

11. The antibody or immunologically active portion thereof of claim 9 or 10 which is selected from the group consisting of:
(a) humanized antibodies; and
(b) chimeric antibodies.

12. The antibody or immunologically active portion thereof of any of claims 9 to 11 which is coupled to a detectable substance, therapeutic moiety or a second antibody.

13. The antibody or immunologically active portion thereof of claim 12 wherein the detectable substance is selected from the group consisting of:
(a) enzymes;
(b) prosthetic groups;
(c) fluorescent materials;
(d) luminescent materials;
(e) bioluminescent materials; and
(f) radioactive materials.

14. The antibody or immunologically active portion thereof of claim 12 wherein the therapeutic moiety is selected from the group consisting of:
(a) cytotoxins;
(b) therapeutic agents; and
(c) radioactive metal ions.

15. A method for detecting the presence of a polypeptide of claim 7 in a sample comprising:
a) contacting the sample with an antibody which selectively binds to the polypeptide; and
b) determining whether the antibody binds to the polypeptide in the sample to thereby detect the presence of a polypeptide of claim 7.

16. A kit comprising an antibody of any one of claims 9 to 11 and instructions for use.

17. A method for detecting the presence of a nucleic acid molecule of claims 1 in a sample comprising:
a) contacting the sample with a nucleic acid probe or primer which selectively hybridizes to the nucleic acid molecule; and
b) determining whether the nucleic acid probe or primer binds to a nucleic acid molecule in the sample to thereby detect the presence of a nucleic acid molecule of claim 1 in the sample; optionally wherein the sample comprises mRNA molecules and is contacted with a nucleic acid probe.

18. Use of a nucleic acid probe or primer which selectively hybridizes to a nucleic acid molecule of claim 1 for identifying a nucleic acid molecule of claim 1.

19. A method for identifying a compound which binds to a polypeptide of claim 7 comprising:
a) contacting the polypeptide, or a cell expressing the polypeptide with a test compound; and
b) determining whether the polypeptide binds to the test compound optionally wherein the binding of the test compound to the polypeptide is detected by a method selected from the group consisting of:
i) detection of binding by direct detection of test compound/polypeptide binding;
ii) detection of binding using a competition binding assay; and
iii) detection of binding using an assay for TWIK-8 activity.

20. An in vitro method for modulating the activity of a polypeptide of claim 7 comprising contacting the polypeptide or a cell expressing the polypeptide with an antibody of any one of claims 9 to 11 which binds to the polypeptide in a sufficient concentration to modulate the activity of the polypeptide.

21. An in vitro method for modulating the expresson of a nucleic acid of claim 1 comprising contacting the nucleic acid or a cell expressing the nucleic acid with an antisense nucleic acid or a ribozyme which binds to the nucleic acid thereby modulating the activity of the nucleic acid.

22. A method for identifying a compound which modulates the activity of a polypeptide of claim 7 comprising:
a) contacting a polypeptide of claim 7 with a test compound; and
b) determining the effect of the test compound on the ability of the polypeptide to modulate a potassium channel mediated activity to thereby identify a compound which modulates the activity of the polypeptide.

## Patentansprüche

1. Isoliertes Nucleinsäuremolekül, aus der aus Folgendem bestehenden Gruppe ausgewählt:
(a) Nucleinsäuremolekül, das die Nucleotidsequenz der Seq.-ID Nr. 1 umfasst;
(b) Nucleinsäuremolekül, das die Nucleotidsequenz der Seq.-ID Nr. 3 umfasst;
(c) isoliertes Nucleinsäuremolekül, das für ein Polypeptid kodiert, das die Aminosäuresequenz der Seq.-ID Nr. 2 umfasst;
(d) isoliertes Nucleinsäuremolekül, das für eine natürlich vorkommende Allelvariante eines Polypeptids kodiert, das die Aminosäuresequenz der Seq.-ID Nr. 2 umfasst;
(e) Nucleinsäuremolekül, das eine Nucleotidsequenz, die zu zumindest 95 % mit der Nucleotidsequenz der Seq.-ID Nr. 1 oder 3 identisch ist, oder ein Komplement davon umfasst; und
(f) Nucleinsäuremolekül, das für ein Polypeptid kodiert, welches eine zu zumindest 95 % mit der Aminosäuresequenz der Seq.-ID Nr. 2 identische Aminosäuresequenz umfasst.

2. Isoliertes Nucleinsäuremolekül, das eine Nucleotidsequenz umfasst, die zur Nucleotidsequenz eines Nucleinsäuremoleküls nach Anspruch 1 komplementär ist.

3. Isoliertes Nucleinsäuremolekül, das ein Nucleinsäuremolekül nach Anspruch 1 und eine für ein heterologes Polypeptid kodierende Nucleotidsequenz umfasst.

4. Vektor, ein Nucleinsäuremolekül nach Anspruch 1 umfassend, worin der Vektor gegebenenfalls ein Expressionsvektor ist.

5. Wirtszelle, die mit einem Expressionsvektor nach Anspruch 4 transfiziert ist.

6. Verfahren zur Herstellung eines Polypeptids, umfassend das Kultivieren der Wirtszelle nach Anspruch 5 in einem geeigneten Kulturmedium, um so das Polypeptid herzustellen.

7. Isoliertes Polypeptid, aus der aus Folgendem bestehenden Gruppe ausgewählt:
a) natürlich vorkommende Allelvariante eines Polypeptids, welche die Aminosäuresequenz der Seq.-ID Nr. 2 umfasst, worin für das Polypeptid ein Nucleinsäuremolekül kodiert, das unter stringenten Bedingungen an ein Nucleinsäuremolekül hybridisiert, das aus Seq.-ID Nr. 1 oder 3 besteht;
b) Polypeptid, für das ein Nucleinsäuremolekül kodiert, das eine zu zumindest 95 % mit einer die Nucleotidsequenz der Seq.-ID Nr. 1 oder 3 umfassenden Nucleinsäure identische Nucleotidsequenz umfasst, und worin das Polypeptid eine Aminosäuresequenz umfasst, die zu zumindest 95 % mit der Aminosäure der Seq.-ID Nr. 2 identisch ist;
c) Polypeptid, das eine Aminosäuresequenz umfasst, die zu zumindest 95 % mit der Aminosäuresequenz der Seq.-ID Nr. 2 identisch ist; und
d) Polypeptid, das die Aminosäuresequenz der Seq.-ID Nr. 2 umfasst.

8. Polypeptid nach Anspruch 7, weiters heterologe Aminosäuresequenzen umfassend.

9. Antikörper oder immunologisch aktiver Teil davon, der selektiv an ein Polypeptid nach Anspruch 7 bindet.

10. Antikörper oder immunologisch aktiver Teil davon nach Anspruch 9, der aus der aus Folgendem bestehenden Gruppe ausgewählt ist:
(a) polyklonale Antikörper;
(b) monoklonale Antikörper;
(c) F(ab)-Fragmente; und
(d) F(ab')2-Fragmente.

11. Antikörper oder immunologisch aktiver Teil davon nach Anspruch 9 oder 10, der aus der aus Folgendem bestehenden Gruppe ausgewählt ist:
(a) humanisierte Antikörper; und
(b) chimäre Antikörper.

12. Antikörper oder immunologisch aktiver Teil davon nach einem der Ansprüche 9 bis 11, der an eine detektierbare Substanz, therapeutische Gruppierung oder einen zweiten Antikörper gebunden ist.

13. Antikörper oder immunologisch aktiver Teil davon nach Anspruch 12, worin die detektierbare Substanz aus der aus Folgendem bestehenden Gruppe ausgewählt ist:
(a) Enzyme;
(b) prosthetische Gruppen;
(c) fluoreszierende Materialien;
(d) lumineszierende Materialien;
(e) biolumineszierende Materialien; und
(f) radioaktive Materialien.

14. Antikörper oder immunologisch aktiver Teil davon nach Anspruch 12, worin die therapeutische Gruppierung aus der aus Folgendem bestehenden Gruppe ausgewählt ist:
(a) Zytotoxine;
(b) therapeutische Wirkstoffe; und
(c) radioaktive Metallionen.

15. Verfahren zur Detektion der Gegenwart eines Polypeptids nach Anspruch 7 in einer Probe, umfassend:
a) das Kontaktieren der Probe mit einem Antikörper, der selektiv an das Polypeptid bindet; und
b) das Bestimmen, ob der Antikörper an das Polypeptid in der Probe bindet, um die Gegenwart eines Polypeptids nach Anspruch 7 zu detektieren.

16. Set, einen Antikörper nach einem der Ansprüche 9 bis 11 und Gebrauchsanleitungen umfassend.

17. Verfahren zur Detektion der Gegenwart eines Nucleinsäuremoleküls nach Anspruch 1 in einer Probe, umfassend:
a) das Kontaktieren der Probe mit einer Nucleinsäuresonde oder einem Primer, die/der selektiv an das Nucleinsäuremolekül hybridisiert; und
b) das Bestimmen, ob die Nucleinsäuresonde oder der Primer an ein Nucleinsäuremolekül in der Probe bindet, um so die Gegenwart eines Nucleinsäuremoleküls nach Anspruch 1 in der Probe zu detektieren; worin die Probe gegebenenfalls mRNA-Moleküle umfasst und mit einer Nucleinsäuresonde kontaktiert wird.

18. Verwendung einer Nucleinsäuresonde oder eines Primers, die/der selektiv an ein Nucleinsäuremolekül nach Anspruch 1 hybridisiert, um ein Nucleinsäuremolekül nach Anspruch 1 zu identifizieren.

19. Verfahren zur Identifikation einer Verbindung, die an ein Polypeptid nach Anspruch 7 hybridisiert, umfassend:
a) das Kontaktieren des Polypeptids oder einer Zelle, die das Polypeptid exprimiert, mit einer Testverbindung; und
b) das Bestimmen, ob das Polypeptid an die Testverbindung bindet, worin die Bindung zwischen der Testverbindung und dem Polypeptid gegebenenfalls durch ein Verfahren detektiert wird, das aus der aus Folgendem bestehenden Gruppe ausgewählt ist:
i) Detektion einer Bindung durch direkte Detektion einer Testverbindung/Polypeptid-Bindung;
ii) Detektion einer Bindung unter Einsatz eines Konkurrenzbindungstests; und
iii) Detektion einer Bindung unter Einsatz eines Tests für TWIK-8-Aktivität.

20. In-vitro-Verfahren zur Modulation der Aktivität eines Polypeptids nach Anspruch 7, umfassend das Kontaktieren des Polypeptids oder einer Zelle, die das Polypeptid exprimiert, mit einem Antikörper nach einem der Ansprüche 9 bis 11, der an das Polypeptid bindet, in ausreichender Konzentration, um die Aktivität des Polypeptids zu modulieren.

21. In-vitro-Verfahren zur Modulation der Expression einer Nucleinsäure nach Anspruch 1, umfassend das Kontaktieren der Nucleinsäure oder einer Zelle, welche die Nucleinsäure exprimiert, mit einer Antisense-Nucleinsäure oder einem Ribozym, die/das an die Nucleinsäure bindet, um so die Aktivität der Nucleinsäure zu modulieren.

22. Verfahren zur Identifikation einer Verbindung, welche die Aktivität eines Polypeptids nach Anspruch 7 moduliert, umfassend:
a) das Kontaktieren eines Polypeptids nach Anspruch 7 mit einer Testverbindung; und
b) das Bestimmen der Wirkung der Testverbindung auf die Fähigkeit des Polypeptids, eine kaliumkanalvermittelte Aktivität zu modulieren, um so eine Verbindung zu identifizieren, welche die Aktivität des Polypeptids moduliert.

## Revendications

1. Molécule isolée d'acide nucléique choisie parmi le groupe constitué de :
(a) une molécule d'acide nucléique comprenant la séquence des nucléotides exposée dans la SEQ ID NO:1;
(b) une molécule d'acide nucléique comprenant la séquence des nucléotides exposée dans la SEQ ID NO: 3 ;
(c) une molécule isolée d'acide nucléique qui code pour un polypeptide comprenant la séquence des acides aminés exposée dans la SEQ ID NO:2 ;
(d) une molécule isolée d'acide nucléique qui code pour un variant allélique d'origine naturelle d'un polypeptide comprenant la séquence des acides aminés exposée dans la SEQ ID NO:2 ;
(e) une molécule d'acide nucléique comprenant une séquence des nucléotides qui est au moins identique à 95% à la séquence des nucléotides de la SEQ ID NO:1 ou 3, ou un complément de celle-ci ;
(f) une molécule d'acide nucléique qui code un polypeptide comprenant une séquence des acides nucléiques au moins identique à 95% à la séquence des acides aminés de la SEQ ID NO:2.

2. Molécule isolée d'acides nucléiques qui comprend une séquence des nucléotides qui est complémentaire à la séquence des nucléotides de la molécule d'acide nucléique selon la revendication 1.

3. Molécule isolée d'acide nucléique comprenant la molécule d'acide nucléique selon la revendication 1 et une séquence des nucléotides codant pour un polypeptide hétérologue.

4. Vecteur comprenant la molécule d'acide nucléique selon la revendication 1, facultativement où le vecteur est un vecteur d'expression.

5. Cellule hôte transfectée avec le vecteur d'expression selon la revendication 4.

6. Procédé de production d'un polypeptide comprenant la mise en culture de la cellule hôte selon la revendication 5 dans un milieu de culture approprié pour produire de cette façon le polypeptide.

7. Polypeptide isolé choisi parmi le groupe constitué de :
(a) un variant allélique d'origine naturelle d'un polypeptide comprenant la séquence des acides aminés de la SEQ ID NO:2, dans lequel le polypeptide est codé par une molécule d'acide nucléique qui est hybridée avec une molécule d'acide nucléique de la SEQ ID NO:1 ou 3 sous des conditions stringentes ;
(b) un polypeptide qui est codé par une molécule d'acide nucléique comprenant une séquence des nucléotides qui est au moins identique à 95% à un acide nucléique comprenant la séquence des nucléotides de la SEQ ID NO:1 ou 3, et où le polypeptide comprend une séquence des acides aminés qui est au moins identique à 95% à l'acide aminé de la SEQ ID NO:2 ;
(c) un polypeptide comprenant une séquence des acides aminés qui est au moins identique à 95% à la séquence des acides aminés de la SEQ ID NO:2 ; et
(d) un polypeptide comprenant la séquence des acides aminés de la SEQ ID NO:2.

8. Polypeptide selon la revendication 7 comprenant en plus des séquences hétérologues des acides aminés.

9. Anticorps, ou partie immunologiquement active de celui-ci, qui se lie sélectivement à un polypeptide selon la revendication 7.

10. Anticorps, ou partie immunologiquement active de celui-ci, selon la revendication 9 qui est choisi parmi le groupe constitué :
(a) d'anticorps polyclonaux ;
(b) d'anticorps monoclonaux ;
(c) de fragments F(ab) ; et
(d) de fragments F(ab')2.

11. Anticorps, ou partie immunologiquement active de celui-ci, selon la revendication 9 ou 10 qui est choisi parmi le groupe constitué :
(a) d'anticorps humanisés ; et
(b) d'anticorps chimériques.

12. Anticorps, ou partie immunologiquement active de celui-ci, selon l'une quelconque des revendications 9 à 11 qui couplé à une substance détectable, une partie thérapeutique ou un second anticorps.

13. Anticorps, ou partie immunologiquement active de celui-ci, selon la revendication 12 dans lequel la substance détectable est choisie parmi le groupe constitué :
(a) d'enzymes ;
(b) de groupes prosthétiques ;
(c) de matériaux fluorescents ;
(d) de matériaux luminescents ;
(e) de matériaux bioluminescents ; et
(f) de matériaux radioactifs.

14. Anticorps, ou partie immunologiquement active de celui-ci, selon la revendication 12 dans lequel la partie thérapeutique est choisie parmi le groupe constitué :
(a) de cytotoxines ;
(b) d'agents thérapeutiques ; et
(c) d'ions métalliques radioactifs.

15. Procédé de détection de la présence d'un polypeptide selon la revendication 7 dans un échantillon comprenant :
(a) la mise en contact de l'échantillon avec un anticorps qui se lie sélectivement au polypeptide ; et
(b) la détermination si l'anticorps se lie au polypeptide dans l'échantillon pour détecter de cette façon la présence d'un polypeptide selon la revendication 7.

16. Trousse comprenant un anticorps selon l'une quelconque des revendications 9 à 11 et les instructions d'utilisation.

17. Procédé de détection de la présence d'une molécule d'acide nucléique selon la revendication 1 dans un échantillon comprenant :
(a) la mise en contact de l'échantillon avec une sonde ou une amorce d'acide nucléique qui est hybridée sélectivement avec la molécule d'acide nucléique ; et
(b) la détermination si la sonde ou de l'amorce d'acide nucléique se lie à une molécule d'acide nucléique dans l'échantillon pour détecter de cette façon la présence d'une molécule d'acide nucléique selon la revendication 1 ; facultativement où l'échantillon comprend des molécules d'ARNm et est mis en contact avec une sonde d'acide nucléique.

18. Utilisation d'une sonde ou amorce d'acide nucléique qui est hybridée sélectivement avec une molécule d'acide nucléique selon la revendication 1 pour l'identification d'une molécule d'acide nucléique selon la revendication 1.

19. Procédé d'identification d'un composé qui se lie à un polypeptide selon la revendication 7 comprenant :
(a) la mise en contact du polypeptide, ou d'une cellule exprimant le polypeptide avec un composé test, et
(b) la détermination si le polypeptide se lie avec le composé test facultativement où la liaison du composé test au polypeptide est détectée par un procédé choisi parmi le groupe constitué de :
i) la détection de la liaison par détection directe de la liaison composé test/polypeptide ;
ii) la détection de la liaison en utilisant un test de liaison par compétition ; et
iii) la détection de la liaison en utilisant un test pour l'activité de TWIK-8.

20. Procédé in vitro de modulation de l'activité d'un polypeptide selon la revendication 7 comprenant la mise en contact du polypeptide ou d'une cellule exprimant le polypeptide avec un anticorps selon l'une quelconque des revendications 9 à 11 qui se lie au polypeptide dans une concentration suffisante pour moduler l'activité du polypeptide.

21. Procédé in vitro de modulation de l'expression d'un acide nucléique selon la revendication 1 comprenant la mise en contact de l'acide nucléique ou d'une cellule exprimant l'acide nucléique avec un acide nucléique antisens ou un ribozyme qui se lie à l'acide nucléique en modulant de cette façon l'activité de l'acide nucléique.

22. Procédé d'identification d'un composé qui module l'activité d'un polypeptide selon la revendication 7 comprenant :
(a) la mise en contact d'un polypeptide selon la revendication 7 avec un composé test ; et
(b) la détermination de l'effet du composé test sur la capacité du polypeptide à moduler une activité médiée par les canaux potassiques pour identifier de cette façon un composé qui module l'activité du polypeptide.


### SEQUENCE LISTING

<110> Millennium Pharmaceuticals, Inc.
<120> 12303, A NOVEL HUMAN TWIK MOLECULE AND USES THEREOF
<130> MNI-142PC
<140>
   <141>
<150> 60/195,734
   <151> 2000-04-07
<160> 3
<170> PatentIn Ver. 2.0
<210> 1
   <211> 1408
   <212> DNA
   <213> Homo sapiens
<220>
   <221> CDS
   <222> (84)..(1340)
<400> 1
   ccacgcgtcc gcggacgcgt gggtcggcac cggcgaggcc gtgctggaac ccgggcctca 60
   gccgcagccg cagcggggcc gac atg acg aca gct ccc cag gag ccc ccc gcc 113
                             Met Thr Thr Ala Pro Gln Glu Pro Pro Ala
                             1               5                   10
   cgg ccc ctc cag gcg ggc agt gga gct ggc ccg gcg cct ggg cgc gcc   161
   Arg Pro Leu Gln Ala Gly Ser Gly Ala Gly Pro Ala Pro Gly Arg Ala
                   15                  20                  25
   atg cgc agc acc acg ctc ctg gcc ctg ctg gcg ctg gtc ttg ctt tac   209
   Met Arg Ser Thr Thr Leu Leu Ala Leu Leu Ala Leu Val Leu Leu Tyr
               30                  35                  40
   ttg gtg tct ggt gcc ctg gtg ttc cgg gcc ctg gag cag ccc cac gag   257
   Leu Val Ser Gly Ala Leu Val Phe Arg Ala Leu Glu Gln Pro His Glu
           45                  50                  55
   cag cag gcc cag agg gag ctg ggg gag gtc cga gag aag ttc ctg agg   305
   Gln Gln Ala Gln Arg Glu Leu Gly Glu Val Arg Glu Lys Phe Leu Arg
       60                  65                  70
   gcc cat ccg tgt gtg agc gac cag gag ctg ggc ctc ctc atc aag gag   353
   Ala His Pro Cys Val Ser Asp Gln Glu Leu Gly Leu Leu Ile Lys Glu
   75                  80                  85                  90
   gtg gct gat gcc ctg gga ggg ggt gcg gac cca gaa acc aac tcg acc   401
   Val Ala Asp Ala Leu Gly Gly Gly Ala Asp Pro Glu Thr Asn Ser Thr
                   95                  100                 105
   agc aac agc agc cac tca gcc tgg gac ctg ggc agc gcc ttc ttt ttc   449
   Ser Asn Ser Ser His Ser Ala Trp Asp Leu Gly Ser Ala Phe Phe Phe
               110                 115                 120
   tca ggg acc atc atc acc acc atc ggc tat ggc aat gtg gcc ctg cgc 497
   Ser Gly Thr Ile Ile Thr Thr Ile Gly Tyr Gly Asn Val Ala Leu Arg
   125 130 135
   aca gat gcc ggg cgc ctc ttc tgc atc ttt tat gcg ctg gtg ggg att 545
   Thr Asp Ala Gly Arg Leu Phe Cys Ile Phe Tyr Ala Leu Val Gly Ile
   140 145 150
   ccg ctg ttt ggg atc cta ctg gca ggg gtc ggg gac cgg ctg ggc tcc 593
   Pro Leu Phe Gly Ile Leu Leu Ala Gly Val Gly Asp Arg Leu Gly Ser
   155 160 165 170
   tcc ctg cgc cat ggc atc ggt cac att gaa gcc atc ttc ttg aag tgg 641
   Ser Leu Arg His Gly Ile Gly His Ile Glu Ala Ile Phe Leu Lys Trp
   175 180 185
   cac gtg cca ccg gag cta gta aga gtg ctg tcg gcg atg ctt ttc ctg 689
   His Val Pro Pro Glu Leu Val Arg Val Leu Ser Ala Met Leu Phe Leu
   190 195 200
   ctg atc ggc tgc ctg ctc ttt gtc ctc acg ccc acg ttc gtg ttc tgc 737
   Leu Ile Gly Cys Leu Leu Phe Val Leu Thr Pro Thr Phe Val Phe Cys
   205 210 215
   tat atg gag gac tgg agc aag ctg gag gcc atc tac ttt gtc ata gtg 785
   Tyr Met Glu Asp Trp Ser Lys Leu Glu Ala Ile Tyr Phe Val Ile Val
   220 225 230
   acg ctt acc acc gtg ggc ttt ggc gac tat gtg gcc ggc gcg gac ccc 833
   Thr Leu Thr Thr Val Gly Phe Gly Asp Tyr Val Ala Gly Ala Asp Pro
   235 240 245 250
   agg cag gac tcc ccg gcc tat cag ccg ctg gtg tgg ttc tgg atc ctg 881
   Arg Gln Asp Ser Pro Ala Tyr Gln Pro Leu Val Trp Phe Trp Ile Leu
   255 260 265
   ctc ggc ctg gct tac ttc gcc tca gtg ctc acc acc atc ggg aac tgg 929
   Leu Gly Leu Ala Tyr Phe Ala Ser Val Leu Thr Thr Ile Gly Asn Trp
   270 275 280
   ctg cga gta gtg tcc cgc cgc act cgg gca gag atg ggc ggc ctc acg 977
   Leu Arg Val Val Ser Arg Arg Thr Arg Ala Glu Met Gly Gly Leu Thr
   285 290 295
   gct cag gct gcc agc tgg act ggc aca gtg aca gcg cgc gtg acc cag 1025
   Ala Gln Ala Ala Ser Trp Thr Gly Thr Val Thr Ala Arg Val Thr Gln
   300 305 310
   cga gcc ggg ccc gcc gcc ccg ccg ccg gag aag gag cag cca ctg ctg 1073
   Arg Ala Gly Pro Ala Ala Pro Pro Pro Glu Lys Glu Gln Pro Leu Leu
   315 320 325 330
   cct cca ccg ccc tgt cca gcg cag ccg ctg ggc agg ccc cga tcc cct 1121
   Pro Pro Pro Pro Cys Pro Ala Gln Pro Leu Gly Arg Pro Arg Ser Pro
   335 340 345
   tcg ccc ccc gag aag gct cag ccg cct tcc ccg ccc acg gcc tcg gcc 1169
   Ser Pro Pro Glu Lys Ala Gln Pro Pro Ser Pro Pro Thr Ala Ser Ala
   350 355 360
   ctg gat tat ccc agc gag aac ctg gcc ttc atc gac gag tcc tcg gat     1217
   Leu Asp Tyr Pro Ser Glu Asn Leu Ala Phe Ile Asp Glu Ser Ser Asp
           365                 370                 375
   acg cag agc gag cgc ggc tgc ccg ctg ccc cgc gcg ccg aga ggt cgc     1265
   Thr Gln Ser Glu Arg Gly Cys Pro Leu Pro Arg Ala Pro Arg Gly Arg
       380                 385                 390
   cgc cgc cca aat ccc ccc agg aag ccc gtg cgg ccc cgc ggc ccc ggg     1313
   Arg Arg Pro Asn Pro Pro Arg Lys Pro Val Arg Pro Arg Gly Pro Gly
   395                 400                 405                 410
   cgt ccc cga gac aaa ggc gtg ccg gtg taggggcagg atccctggcc           1360
   Arg Pro Arg Asp Lys Gly Val Pro Val
                   415
   gggcctctca agggcttcgt ttctgctctc cccggcatgc ctggcttc                1408
<210> 2
   <211> 419
   <212> PRT
   <213> Homo sapiens
<400> 2
   Met Thr Thr Ala Pro Gln Glu Pro Pro Ala Arg Pro Leu Gln Ala Gly
    1               5                  10                  15
   Ser Gly Ala Gly Pro Ala Pro Gly Arg Ala Met Arg Ser Thr Thr Leu
               20                  25                  30
   Leu Ala Leu Leu Ala Leu Val Leu Leu Tyr Leu Val Ser Gly Ala Leu
           35                  40                  45
   Val Phe Arg Ala Leu Glu Gln Pro His Glu Gln Gln Ala Gln Arg Glu
       50                  55                  60
   Leu Gly Glu Val Arg Glu Lys Phe Leu Arg Ala His Pro Cys Val Ser
   65                  70                  75                  80
   Asp Gln Glu Leu Gly Leu Leu Ile Lys Glu Val Ala Asp Ala Leu Gly
                   85                  90                  95
   Gly Gly Ala Asp Pro Glu Thr Asn Ser Thr Ser Asn Ser Ser His Ser
               100                 105                 110
   Ala Trp Asp Leu Gly Ser Ala Phe Phe Phe Ser Gly Thr Ile Ile Thr
           115                 120                 125
   Thr Ile Gly Tyr Gly Asn Val Ala Leu Arg Thr Asp Ala Gly Arg Leu
       130                 135                 140
   Phe Cys Ile Phe Tyr Ala Leu Val Gly Ile Pro Leu Phe Gly Ile Leu
   145                 150                 155                 160
   Leu Ala Gly Val Gly Asp Arg Leu Gly Ser Ser Leu Arg His Gly Ile
                   165                 170                 175
   Gly His Ile Glu Ala Ile Phe Leu Lys Trp His Val Pro Pro Glu Leu
               180                 185                 190
   Val Arg Val Leu Ser Ala Met Leu Phe Leu Leu Ile Gly Cys Leu Leu
           195                 200                 205
   Phe Val Leu Thr Pro Thr Phe Val Phe Cys Tyr Met Glu Asp Trp Ser
       210                 215                 220
   Lys Leu Glu Ala Ile Tyr Phe Val Ile Val Thr Leu Thr Thr Val Gly
   225                 230                 235                 240
   Phe Gly Asp Tyr Val Ala Gly Ala Asp Pro Arg Gln Asp Ser Pro Ala
                   245                 250                 255
   Tyr Gln Pro Leu Val Trp Phe Trp Ile Leu Leu Gly Leu Ala Tyr Phe
               260                 265                 270
   Ala Ser Val Leu Thr Thr Ile Gly Asn Trp Leu Arg Val Val Ser Arg
           275                 280                 285
   Arg Thr Arg Ala Glu Met Gly Gly Leu Thr Ala Gln Ala Ala Ser Trp
       290                 295                 300
   Thr Gly Thr Val Thr Ala Arg Val Thr Gln Arg Ala Gly Pro Ala Ala
   305                 310                 315                 320
   Pro Pro Pro Glu Lys Glu Gln Pro Leu Leu Pro Pro Pro Pro Cys Pro
                   325                 330                 335
   Ala Gln Pro Leu Gly Arg Pro Arg Ser Pro Ser Pro Pro Glu Lys Ala
               340                 345                 350
   Gln Pro Pro Ser Pro Pro Thr Ala Ser Ala Leu Asp Tyr Pro Ser Glu
           355                 360                 365
   Asn Leu Ala Phe Ile Asp Glu Ser Ser Asp Thr Gln Ser Glu Arg Gly
       370                 375                 380
   Cys Pro Leu Pro Arg Ala Pro Arg Gly Arg Arg Arg Pro Asn Pro Pro
   385                 390                 395                 400
   Arg Lys Pro Val Arg Pro Arg Gly Pro Gly Arg Pro Arg Asp Lys Gly
                   405                 410                 415
   Val Pro Val
<210> 3
   <211> 1257
   <212> DNA
   <213> Homo sapiens
<220>
   <221> CDS
   <222> (1)..(1257)
<400> 3
   atg acg aca gct ccc cag gag ccc ccc gcc cgg ccc ctc cag gcg ggc    48
   Met Thr Thr Ala Pro Gln Glu Pro Pro Ala Arg Pro Leu Gln Ala Gly
    1               5                  10                  15
   agt gga gct ggc ccg gcg cct ggg cgc gcc atg cgc agc acc acg ctc    96
   Ser Gly Ala Gly Pro Ala Pro Gly Arg Ala Met Arg Ser Thr Thr Leu
               20                  25                  30
   ctg gcc ctg ctg gcg ctg gtc ttg ctt tac ttg gtg tct ggt gcc ctg    144
   Leu Ala Leu Leu Ala Leu Val Leu Leu Tyr Leu Val Ser Gly Ala Leu
           35                  40                  45
   gtg ttc cgg gcc ctg gag cag ccc cac gag cag cag gcc cag agg gag    192
   Val Phe Arg Ala Leu Glu Gln Pro His Glu Gln Gln Ala Gln Arg Glu
       50                  55                  60
   ctg ggg gag gtc cga gag aag ttc ctg agg gcc cat ccg tgt gtg agc    240
   Leu Gly Glu Val Arg Glu Lys Phe Leu Arg Ala His Pro Cys Val Ser
   65                  70                  75                  80
   gac cag gag ctg ggc ctc ctc atc aag gag gtg gct gat gcc ctg gga    288
   Asp Gln Glu Leu Gly Leu Leu Ile Lys Glu Val Ala Asp Ala Leu Gly
                   85                  90                  95
   ggg ggt gcg gac cca gaa acc aac tcg acc agc aac agc agc cac tca    336
   Gly Gly Ala Asp Pro Glu Thr Asn Ser Thr Ser Asn Ser Ser His Ser
               100                 105                 110
   gcc tgg gac ctg ggc agc gcc ttc ttt ttc tca ggg acc atc atc acc    384
   Ala Trp Asp Leu Gly Ser Ala Phe Phe Phe Ser Gly Thr Ile Ile Thr
           115                 120                 125
   acc atc ggc tat ggc aat gtg gcc ctg cgc aca gat gcc ggg cgc ctc    432
   Thr Ile Gly Tyr Gly Asn Val Ala Leu Arg Thr Asp Ala Gly Arg Leu
       130                 135                 140
   ttc tgc atc ttt tat gcg ctg gtg ggg att ccg ctg ttt ggg atc cta    480
   Phe Cys Ile Phe Tyr Ala Leu Val Gly Ile Pro Leu Phe Gly Ile Leu
   145                 150                 155                 160
   ctg gca ggg gtc ggg gac cgg ctg ggc tcc tcc ctg cgc cat ggc atc    528
   Leu Ala Gly Val Gly Asp Arg Leu Gly Ser Ser Leu Arg His Gly Ile
                   165                 170                 175
   ggt cac att gaa gcc atc ttc ttg aag tgg cac gtg cca ccg gag cta    576
   Gly His Ile Glu Ala Ile Phe Leu Lys Trp His Val Pro Pro Glu Leu
               180                 185                 190
   gta aga gtg ctg tcg gcg atg ctt ttc ctg ctg atc ggc tgc ctg ctc    624
   Val Arg Val Leu Ser Ala Met Leu Phe Leu Leu Ile Gly Cys Leu Leu
           195                 200                 205
   ttt gtc ctc acg ccc acg ttc gtg ttc tgc tat atg gag gac tgg agc    672
   Phe Val Leu Thr Pro Thr Phe Val Phe Cys Tyr Met Glu Asp Trp Ser
       210                 215                 220
   aag ctg gag gcc atc tac ttt gtc ata gtg acg ctt acc acc gtg ggc    720
   Lys Leu Glu Ala Ile Tyr Phe Val Ile Val Thr Leu Thr Thr Val Gly
   225                 230                 235                 240
   ttt ggc gac tat gtg gcc ggc gcg gac ccc agg cag gac tcc ccg gcc    768
   Phe Gly Asp Tyr Val Ala Gly Ala Asp Pro Arg Gln Asp Ser Pro Ala
                   245                 250                 255
   tat cag ccg ctg gtg tgg ttc tgg atc ctg ctc ggc ctg gct tac ttc    816
   Tyr Gln Pro Leu Val Trp Phe Trp Ile Leu Leu Gly Leu Ala Tyr Phe
               260                 265                 270
   gcc tca gtg ctc acc acc atc ggg aac tgg ctg cga gta gtg tcc cgc      864
   Ala Ser Val Leu Thr Thr Ile Gly Asn Trp Leu Arg Val Val Ser Arg
           275                 280                 285
   cgc act cgg gca gag atg ggc ggc ctc acg gct cag gct gcc agc tgg      912
   Arg Thr Arg Ala Glu Met Gly Gly Leu Thr Ala Gln Ala Ala Ser Trp
       290                 295                 300
   act ggc aca gtg aca gcg cgc gtg acc cag cga gcc ggg ccc gcc gcc      960
   Thr Gly Thr Val Thr Ala Arg Val Thr Gln Arg Ala Gly Pro Ala Ala
   305                 310                 315                 320
   ccg ccg ccg gag aag gag cag cca ctg ctg cct cca ccg ccc tgt cca      1008
   Pro Pro Pro Glu Lys Glu Gln Pro Leu Leu Pro Pro Pro Pro Cys Pro
                   325                 330                 335
   gcg cag ccg ctg ggc agg ccc cga tcc cct tcg ccc ccc gag aag gct      1056
   Ala Gln Pro Leu Gly Arg Pro Arg Ser Pro Ser Pro Pro Glu Lys Ala
               340                 345                 350
   cag ccg cct tcc ccg ccc acg gcc tcg gcc ctg gat tat ccc agc gag      1104
   Gln Pro Pro Ser Pro Pro Thr Ala Ser Ala Leu Asp Tyr Pro Ser Glu
           355                 360                 365
   aac ctg gcc ttc atc gac gag tcc tcg gat acg cag agc gag cgc ggc      1152
   Asn Leu Ala Phe Ile Asp Glu Ser Ser Asp Thr Gln Ser Glu Arg Gly
       370                 375                 380
   tgc ccg ctg ccc cgc gcg ccg aga ggt cgc cgc cgc cca aat ccc ccc      1200
   Cys Pro Leu Pro Arg Ala Pro Arg Gly Arg Arg Arg Pro Asn Pro Pro
   385                 390                 395                 400
   agg aag ccc gtg cgg ccc cgc ggc ccc ggg cgt ccc cga gac aaa ggc      1248
   Arg Lys Pro Val Arg Pro Arg Gly Pro Gly Arg Pro Arg Asp Lys Gly
                   405                 410                 415
   gtg ccg gtg                                                          1257
   Val Pro Val